# EUROPEAN PATENT APPLICATION

(11) **EP 3 855 183 A1**
(43) Date of publication of application: **28.07.2021**
(21) Application number: 20305060.4
(22) Date of filing: 24.01.2020
(51) Int. Cl.: G01N 33/569, C07K 14/435, G01N 33/68

(54) **IDENTIFICATION AND SYNTHESIS OF DRUG CANDIDATES DERIVED FROM HUMAN MICROBIOME METASECRETOME PROTEINS**

(71) Applicant: Enterome S.A., 75011 Paris (FR)
(72) Inventor: CHENE, Laurent, 45170 Neuville aux Bois (FR); STROZZI, Francesco, 75003 Paris (FR); BONNY, Christophe, 75011 Paris (FR)
(74) Representative: Office Freylinger

(57) **Abstract**

The present invention relates to the treatment of diseases relating to proteins of the human microbiome metasecretome and, thus, to microbiome interactions, especially microbiome-host interactions. In particular the present invention relates to a method for identification of secreted peptides and proteins of the human microbiome. The present invention also relates to methods for generating a database of human microbiome metasecretome protein sequences. Furthermore, the present invention relates to a method for preparing a protein of the human microbiome metasecretome as well as to the use of such proteins in medicine.

SEQUENCE LISTING

<110>  ENTEROME SA

<120>  IDENTIFICATION AND SYNTHESIS OF DRUG CANDIDATES DERIVED FROM
       HUMAN MICROBIOME METASECRETOME PROTEINS

<130>  P-GRAF-006/EP

<160>  10

<170>  PatentIn version 3.5

<210>  1
<211>  121
<212>  PRT
<213>  microbiota

<400>  1

Ala Phe Leu Phe Thr Ser Thr Gly Val Pro Lys Lys Ala Ala Glu Ala
1               5                   10                  15

Ala Phe Phe Leu Tyr Leu Asn Lys Gly Thr Lys Lys Gly Ser Arg Ser
            20                  25                  30

Cys Phe Phe Ile Tyr Leu Asp Arg Gly Thr Lys Lys Gly Ser Arg Ser
        35                  40                  45

Cys Phe Phe Tyr Leu Pro Arg Gln Gly Tyr Gln Lys Gly Ser Arg Gly
    50                  55                  60

Cys Phe Phe Ile Tyr Leu Asp Arg Gly Thr Lys Lys Gly Ser Arg Gly
65                  70                  75                  80

Cys Phe Phe Ile Tyr Leu Asp Cys Glu Lys Arg Ala Gly Asn Val Cys
                85                  90                  95

Ile Arg Lys Cys Arg Gly Arg Tyr Leu His Lys Lys Thr Pro Arg Arg
            100                 105                 110

Tyr Arg Asn Ala Glu Ala Thr Cys Ser
        115                 120

<210>  2
<211>  100
<212>  PRT
<213>  microbiota

<400>  2

Gln Thr Arg Lys Gln Arg Glu Asp Ala Lys Arg Glu Ala Trp Lys Lys
1               5                   10                  15

Glu Arg Lys Glu Lys Lys Ala Leu Glu Ala Gln Gln Asp Ser Val Ser
            20                  25                  30

Phe Met Lys Asp Thr Glu Ser Cys Cys Ala Ser Lys Ala Phe Phe Ser
        35                  40                  45

Leu Arg Ser Phe Phe His Ala Ser Arg Leu Ala Ser Ser Leu Cys Phe
    50                  55                  60

Leu Val Cys Ala Phe Val Thr Pro Leu Lys Gln Thr Ser Asn Asn Ala
65                  70                  75                  80

Ala Lys Asn Asn Thr Phe Phe Ile Ile Lys Ala Val Leu Leu Ile Asn
                85                  90                  95

Ile Ser Phe Arg
            100

<210> 3
<211> 78
<212> PRT
<213> microbiota

<400> 3

Ala Arg Asn Tyr Thr Cys Asp Val Cys Gly Asn Gly Thr Ile Gln Ile
1               5                   10                  15

Val Ser Ser His Ile Ile His Asn Val His Cys Gly Phe Ile Pro Cys
            20                  25                  30

Asn Lys Ile Asn Gly Val Met Asp Glu Val Val Tyr Lys Thr Val Thr
        35                  40                  45

Glu Asn Asn Glu Ala Cys Asn Asn Cys Gly Val Ser Tyr Thr Tyr Lys
    50                  55                  60

Val Tyr Gly Asp Met Glu Ile Ile Cys Lys Ala Lys Ala Asn
65                  70                  75

<210> 4
<211> 84
<212> PRT
<213> microbiota

<400> 4

Ala Glu Pro Ala Asp Thr Ala Ile Ser Glu Arg Arg Val Glu Leu Cys
1               5                   10                  15

Gly Asn Cys Gly Gly Arg Met Val Thr Ser Thr Thr Trp Gly Ser Trp

            20                  25                  30

Tyr Thr Val Ala Gln Ile Lys Cys Thr His His Asn Tyr Gly Thr Asp
        35                  40                  45

Leu Arg Gln Gln Arg Asp Gly Thr Ala Thr Thr Lys Cys Gln Gly Cys
    50                  55                  60

Gly Gln Gly Tyr Thr Thr Ser Lys Ser Gln Thr Arg Ile Val Cys His
65                  70                  75                  80

Gly Tyr Asp Ser

<210> 5
<211> 144
<212> PRT
<213> microbiota

<400> 5

Ala Ala Phe Val Phe Ser Asn Ser Leu Lys Pro Ala Asn Ala Ser Ser
1               5                   10                  15

Ala Glu Ser Ser Arg Leu Leu Ile His Val Asn Ser Phe Phe Ser Gln
            20                  25                  30

Leu Gly Leu Lys Pro Ile Ser Glu Asn Leu Leu Arg Lys Thr Ala His
        35                  40                  45

Phe Cys Glu Phe Gly Met Leu Gly Ile Leu Ala Ser Ser Ala Cys Ala
    50                  55                  60

Met Phe Ser Gly Ala Tyr Ser Ala Ala Ser Leu Pro Ser Leu Arg Arg
65                  70                  75                  80

Arg Gly Phe Phe Ile Ser Phe Gly Val Ser Val Ala Cys Ala Val Cys
                85                  90                  95

Asp Glu Thr Ile Gln Tyr Phe Val Pro Gly Arg Ala Cys Arg Val Thr
            100                 105                 110

Asp Met Leu Ile Asp Ser Ala Gly Ala Leu Cys Gly Leu Ala Ala Val
        115                 120                 125

Leu Ala Phe Cys Ala Ala Ile Arg Val Arg Arg Arg Arg Arg Arg Asn
    130                 135                 140

<210> 6

<211>  58
<212>  PRT
<213>  microbiota

<400>  6

Leu Ala Gly Pro Gly Ser Gly Cys Arg Phe Thr Pro Ser Cys Ser Thr
1               5                   10                  15

Tyr Phe Ile Gln Ala Val Glu Ile His Gly Ala Leu Lys Gly Ser Leu
            20                  25                  30

Met Gly Ile Trp Arg Ile Leu Arg Cys Asn Pro Trp Gly Gly Cys Gly
        35                  40                  45

Tyr Asp Pro Val Pro Pro Arg Lys Pro Arg
    50                  55

<210>  7
<211>  44
<212>  PRT
<213>  microbiota

<400>  7

Ala Lys Leu Gly Met Ala Ala Gly Ala Met Leu Val Leu Gly Leu Leu
1               5                   10                  15

Ala Ala Gly Ala Ser Gly Gly Thr Leu Ile Leu Ala Ala Leu Ala Leu
            20                  25                  30

Cys Ala Val Thr Leu Leu Cys Gly Arg Lys Lys Gln
        35                  40

<210>  8
<211>  75
<212>  PRT
<213>  microbiota

<400>  8

Val Glu Lys Lys Thr Val Ile Thr Lys Cys Ala Ile Thr Val Asn Glu
1               5                   10                  15

Tyr Arg Glu Lys Val Val Pro Ser Met Arg Lys Ile His Ala Ile Val
            20                  25                  30

Ile Phe Val Ser Tyr Ser Ile Asn His Leu Tyr Lys Asn Cys Glu Pro
        35                  40                  45

Glu Gln Leu Phe Ser Pro Gly Arg Lys Thr Lys Lys Pro Pro Pro Ala
    50                  55                  60

Thr Cys Arg Lys Arg Leu Asn Leu Gln Tyr Phe
65                  70                  75

<210>  9
<211>  96
<212>  PRT
<213>  microbiota

<400>  9

Glu Ile Thr Gln Pro Cys Asn His Val Lys Ser Asp Trp Ile Ile Asp
1               5                   10                  15

Lys Glu Ala Thr Cys Ile Gly Ser Tyr Ala Phe Tyr Asn Cys Thr Ser
            20                  25                  30

Leu Thr Ser Ile Glu Ile Ser Thr Ser Val Thr Lys Ile Lys Tyr Arg
        35                  40                  45

Ala Phe Ala Ser Cys Arg Ala Leu Asn Asn Ile Tyr Tyr Thr Gly Thr
    50                  55                  60

Leu Thr Gln Trp Asn Glu Ile Ser Lys Asp Thr Asn Trp Asn Trp Ala
65                  70                  75                  80

Ala Pro Leu Asn Cys Lys Val Ile Cys Leu Asn Gly Thr Cys Tyr Leu
                85                  90                  95

<210>  10
<211>  98
<212>  PRT
<213>  microbiota

<400>  10

Leu Leu Val Ser Val Cys Thr Ala Ala Gly Leu Leu Ala Val Ala Met
1               5                   10                  15

Arg Gln Ile Glu Pro Leu Leu Ala Trp Leu Arg Thr Leu Glu Val Tyr
            20                  25                  30

Phe Gln Gly Gln Ser Pro Ala Val Leu Leu Arg Ala Leu Gly Ile Ala
        35                  40                  45

Leu Val Ala Gln Phe Ala Ala Asp Thr Cys Arg Glu Ala Gly Leu Cys
    50                  55                  60

Ala Ala Ser Thr Ala Ile Glu Leu Cys Gly Arg Val Leu Val Leu Leu
65                  70                  75                  80

Gln Ala Leu Pro Leu Leu Arg Ser Leu Leu Gly Ser Phe Ala Asp Tyr
                85                  90                  95

Leu Gln

## Description

The present invention relates to microbiome interactions, in particular to microbiome-host interactions, but also to other interactions such bacterial-bacterial interactions. In particular the present invention relates to a method for identification and, optionally, synthesis of secreted peptides and proteins of the human microbiome, which may represent drug candidates. The present invention also relates to methods for generating a database of human microbiome metasecretome protein sequences. Furthermore, the present invention relates to a method for preparing a protein of the human microbiome metasecretome as well as to the use of such proteins in medicine. Proteins of the human microbiome metasecretome may interact with human proteins involved in various diseases and, thus, represent drug candidates for the treatment of such diseases.

The human intestinal microbiota (HIM) is a huge and complex community of 10¹⁴ bacteria which colonize the human gastrointestinal tract (GIT) and it is now considered as a hidden human organ. HIM has essential functions such as maintaining intestinal homeostasis, inhibiting the growth of pathogens, producing antimicrobial compounds and improving the intestinal barrier function. A number of evidences support the fact that several gastrointestinal disorders including inflammatory bowel diseases (IBD), irritable bowel syndrome, nonalcoholic steatohepatitis and alcoholic liver disease and even brain activity (gut brain axis) are the result of an altered balance (dysbiosis) of the intestinal microbiota. More recently, the human microbiota composition has also been associated with effectiveness of antitumoral treatment as chemotherapy and check point inhibitor treatment. Thus, change of the ratio of beneficial vs. neutral or aggressive bacterial species results in altered levels of metabolites and bacterial components normally interacting with the host and contributes to altered gut homeostasis observed in such diseases or therapeutic treatment.

Moreover, the human microbiome plays an important role in inflammation and immunomodulatory responses in the host, although many of the underlying mechanisms are yet to be identified. For instance, recent studies showed the potential of certain bacterial species to activate or inhibit an immune response in the host (Geva-Zatorsky N, Sefik E, Kua L, Pasman L, Tan TG, Ortiz-Lopez A, Yanortsang TB, Yang L, Jupp R, Mathis D, Benoist C, Kasper DL. Mining the Human Gut Microbiota for Immunomodulatory Organisms. Cell. 2017 Feb 23;168(5):928-943.e11). In this study, 53 human microbiome bacterial species have been identified to have a clear immunomodulatory effect following monocolonization of sterile mice.

The above described effects of the human microbiome on the host and the described interactions are mediated by the ensemble of molecules produced and secreted by the microbiome itself, which can interact directly with the human host cells and, in particular, with the immune system receptors. As described by Ratner et al., 2017 (Ratner, D., Orning, M. P. A., & Lien, E. (2017). Bacterial secretion systems and regulation of inflammasome activation. Journal of Leukocyte Biology, 707(1), 165-181), the secreted proteins produced by specific pathogenic bacteria play a key role in this immunomodulatory effect. Ratner et al. propose that a fine balance exists between secretion system-mediated activation and inhibition, which results in a net activation of inflammation control, clearance or spread of a possible infection. Given the co-habitation and thus the selective pressure to which the human microbiome is subject, it is not surprising that those bacteria are involved in an even more fine grained regulation and interaction with the host immune system. As a consequence, these interactions between the microbiome and the host are a key component in the modulation of a number of diseases and immune responses (Kinross, J. M., Darzi, A. W., & Nicholson, J. K. (2011). Gut microbiome-host interactions in health and disease. Genome Medicine, 3(3), 14).

The commensal molecules mediating the interaction with the host are primarily metabolites and proteins, which act on host receptors and lead to specific signaling in the human cells. However, most studies in this field focused so far on identification of commensal metabolites supporting immune system homeostasis or global host metabolism (Nicholson JK, Holmes E, Kinross J, Burcelin R, Gibson G, Jia W, Pettersson S. Host-gut microbiota metabolic interactions. Science. 2012 Jun 8;336(6086):1262-7; Levy M, Thaiss CA, Elinav E. Metabolites: messengers between the microbiota and the immune system. Genes Dev. 2016 Jul 15;30(14):1589-97; Guo CJ, Chang FY, Wyche TP, Backus KM, Acker TM, Funabashi M, Taketani M, Donia MS, Nayfach S, Pollard KS, Craik CS, Cravatt BF, Clardy J, Voigt CA, Fischbach MA. Discovery of Reactive Microbiota-Derived Metabolites that Inhibit Host Proteases. Cell. 2017 Jan 26;168(3):517-526.e18; Cohen LJ, Kang HS, Chu J, Huang YH, Gordon EA, Reddy BV, Ternei MA, Craig JW, Brady SF. Functional metagenomic discovery of bacterial effectors in the human microbiome and isolation of commendamide, a GPCR G2A/132 agonist. Proc Natl Acad Sci USA. 2015 Sep 1;112(35):E4825-34).

Identification of these metabolites is an emerging field that is supported by metabolomics tools and studies in total fecal samples or cultured bacteria. However, identification and use of metabolite that could be associated with therapeutic effect is associated with some hurdles, such as difficulties in compound synthesis and full structure determination, that may require analytical tools as HPLC, LC-MS and NMR and may take a long time (Sharon G, Garg N, Debelius J, Knight R, Dorrestein PC, Mazmanian SK. Specialized metabolites from the microbiome in health and disease. Cell metabolism. 2014;20(5):719-730; Wilson MR, Zha L, Balskus EP. Natural product discovery from the human microbiome. J Biol Chem. 2017 May 26;292(21):8546-8552).

Apart from the microbial metabolites, microbial proteins are also considered as molecules interacting with the host (Weigele BA, Orchard RC, Jimenez A, Cox GW, Alto NM. A systematic exploration of the interactions between bacterial effector proteins and host cell membranes. Nat Commun. 2017 Sep 14;8(1):532; Devin K. Schweppe, Christopher Harding, Juan D. Chavez, Xia Wu, Elizabeth Ramage, Pradeep K. Singh, Colin Manoil, James E. Bruce, Host-Microbe Protein Interactions during Bacterial Infection, Chemistry & Biology, 2015, 22(11):1521-1530; Guven-Maiorov E, Tsai CJ, Nussinov R. Structural host-microbiota interaction networks. PLoS Comput Biol. 2017 Oct 12,13(10):e1005579). Microbial proteins may be able to interact with many human receptors as G-protein coupled receptors (GPCRs), kinase receptor and transporter and may affect many different signaling pathways involved in various functions, such as immune surveillance, metabolism and cellular integrity. Recently, molecular mimicry between human and microbial proteins emerged as proposed mechanism of action of many bacterial proteins. For example, ClpB, a member of a protein-disaggregating multi-chaperone system in *E. coli,* was reported to mimic alpha MSH (Breton J, Tennoune N, Lucas N, Francois M, Legrand R, Jacquemot J, Goichon A, Guérin C, Peltier J, Pestel-Caron M, Chan P, Vaudry D, do Rego JC, Liénard F,Pénicaud L, Fioramonti X, Ebenezer IS, Hökfelt T, Déchelotte P, Fetissov SO. Gut Commensal E. coli Proteins Activate Host Satiety Pathways following Nutrient-Induced Bacterial Growth. Cell Metab. 2016 Feb 9;23(2):324-34); *Helicobacter pylori* CagA was shown to interact with human tumor suppressor TP53BP2 (Buti L, Spooner E, Van der Veen AG, Rappuoli R, Covacci A, Ploegh HL. Helicobacter pylori cytotoxin-associated gene A (CagA) subverts the apoptosis-stimulating protein of p53 (ASPP2) tumor suppressor pathway of the host. Proc Natl Acad Sci U S A. 2011 May 31;108(22):9238-43); SLPA from *Lactobacillus acidophilus* was reported to be a DC-SIGN ligand that is functionally involved in the modulation of DCs and T cells functions; P40 from *L. rhamnosus* was shown to activate EGFR in young adult mouse colon epithelial cells and human colonic epithelial cell line; and the protein FAp2 from *fusobacterium nucleatum* was shown to mediate *fusobacterium nucleatum* colorectal adenocarcinoma enrichment by binding to tumor-expressed Gal-GalNAc, and to bind to the TIGIT receptor (Gur C, Ibrahim Y, Isaacson B, Yamin R, Abed J, Gamliel M, Enk J, Bar-On Y, Stanietsky-Kaynan N, Coppenhagen-Glazer S, Shussman N, Almogy G, Cuapio A, Hofer E, Mevorach D, Tabib A, Ortenberg R, Markel G, Miklić K, Jonjic S, Brennan CA, Garrett WS, Bachrach G, Mandelboim O. Binding of the Fap2 protein of Fusobacterium nucleatum to human inhibitory receptor TIGIT protects tumors from immune cell attack. Immunity. 2015 Feb 17;42(2):344-355). Accordingly, many different proteins from commensal bacteria have the ability to mimic host proteins and, thus, to agonize or antagonize several cellular pathways. Moreover, various proteins from the human microbiome may increase the secretion of host proteins (secretagogues, i.e. (microbiome) substances causing secretion of other (human) substances).

Such microbial proteins may thus be useful in various therapeutic applications. For example, modulation of protein expression levels and expression in specific conditions or diseases can suggest a biological effect on the host. Once identified, microbial proteins can then be tested and validated for their biological effect on appropriate cellular assays, ligand binding assays, and biochemical and enzymatic assays.

Traditionally, cultivation of microorganisms and screening for individual strains with the desired phenotype is widely used. However, even though novel cultivation methods emerged (Sommer MO. Advancing gut microbiome research using cultivation. Curr Opin Microbiol. 2015 Oct;27:127-32), the complex microbial community of the human gut is not culturable by standard laboratory techniques. Accordingly, culture-independent approaches, collectively known as functional metagenomics, represent the major tool for assessing the human microbiome. The recent development of high-throughput sequencing and associated metagenomic techniques opens up new opportunities to begin to understand this complex community. High-throughput screening (HTS) technologies enable the analysis of the entire genome of all microorganisms belonging to an ecological niche, including those which are not cultivable.

In order to identify such microbial proteins interacting with the human host (or other interactions of interest such as bacterial-bacterial interactions), which may be useful in therapeutic applications, a database or library containing proteins of the entire microbiome, which have the potential to interact with the human host, is desired. Accordingly, there is a need for a large library/database of proteins from commensal bacteria. Such a library/database may then be screened for proteins, which are able, for example, to bind to selected human receptors, such as GPCRs or to more generally elicit a specific biological effect on metabolism, immunity, or cellular integrity by using dedicated readouts.

Despite the manifold metagenomics approaches addressing the human gut microbiome, there is no library or database focusing on such human gut microbiome proteins, in particular proteins which are potentially interacting with the human host and, thus, providing targets and/or drug candidates for therapeutic applications.

In view of the above, it is the object of the present invention to provide a method for generating a database of human microbiome proteins, in particular proteins interacting with the human host, such as database of human gut microbiome proteins interacting with the human host. In particular, it is an object of the present invention to provide a method for identifying a peptide or protein of the human microbiome metasecretome, including the human gut microbiome metasecretome. Moreover, it is also an object of the present invention to provide a method for synthesizing such a protein (*in vitro*) and to provide such proteins for medical applications.

These objects are achieved by means of the subject-matter set out below and in the appended claims.

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" *e.g.,* a composition "comprising" X may consist exclusively of X or may include something additional *e.g.,* X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" *e.g*., a composition which is "substantially free" from Y may be completely free from Y. Where necessary, the word "substantially" may be omitted from the definition of the invention.

The term "about" in relation to a numerical value x means x ± 10%.

### ITEMS OF THE INVENTION

The present invention provides in particular the following items:
1. A method for providing a human microbiota protein drug candidate, the method comprising the following steps:
   (i) providing a plurality of human microbiota protein sequences and/or a plurality of nucleic acid sequences encoding a plurality of human microbiota proteins;
   (ii) identifying in the sequences provided in step (i) one or more sequence(s) of (a) human microbiota protein drug candidate(s), wherein the sequence(s) of the human microbiota protein drug candidate(s) is/are selected according to the following criteria:
      - (a) sequence(s) having, or coding for proteins having, a signal peptide;
      - (a) sequence(s) having, or coding for proteins having, a length of 20 - 500 amino acids; and
      - (a) sequence(s) comprising, or coding for (a) protein(s) comprising, at least two cysteine residues and/or a primary and/or a secondary structure element conferring a conformational rigid structure; and
   (iii) preparing one or more human microbiota protein(s) having, or encoded by, the sequence(s) identified in step (ii).
2. The method according to item 1, wherein the microbiota is a microbiota selected from the group consisting of gastrointestinal tract microbiota, lung microbiota, saliva microbiota, seminal fluid microbiota, skin microbiota and vagina microbiota.
3. The method according to item 1 or 2, wherein the microbiota is gastrointestinal tract microbiota selected from gut microbiota and oral cavity microbiota.
4. The method according to any one of items 1 to 3, wherein the human microbiota protein is a bacterial protein, archaea protein, protist protein, fungi protein, virus protein and/or phage protein.
5. The method according to any one of items 1 to 4, wherein the human microbiota protein is a bacterial protein, preferably of the human gastrointestinal tract microbiota metasecretome.
6. The method according to any one of items 1 to 4, wherein the human microbiota protein is a bacterial protein of the human gastrointestinal tract microbiota.
7. The method according to any one of items 1 to 6, wherein in step (i) a database of human microbiota protein sequences and/or a database of nucleic acid sequences encoding human microbiota proteins is provided.
8. The method according to item 7, wherein the microbiota database comprises microbiota sequences of multiple host individuals.
9. The method according to item 8, wherein the microbiota database comprises microbiota data of a single host individual, but not of multiple host individuals.
10. The method according to any one of items 7 - 9, wherein step (i) comprises the following sub-steps:
   (i-a) optionally, identifying microbiota protein sequences or nucleic acid sequences from (a) sample(s) of a single or multiple individual(s), and
   (i-b) compiling a database containing microbiota protein sequences or nucleic acid sequences of a single or multiple individual(s).
11. The method according to item 10, wherein the sample in step (i-a) is a stool sample.
12. The method according to any one of items 1 to 11, wherein the identification of a signal peptide in step (ii) is performed *in silico.*
13. The method according to any one of items 1 to 12, wherein the identification of a signal peptide in step (ii) is performed by using two distinct methods, wherein a sequence having, or coding for, a signal peptide according to both methods is selected.
14. The method according to any one of items 1 to 13, wherein the identification of a signal peptide in step (ii) is performed by using Phobius and/or SignalP.
15. The method according to any one items 1 to 14, wherein the human microbiota protein has a length of 20 - 350 amino acids.
16. The method according to any one items 1 to 15, wherein the human microbiota protein comprises at least two cysteine residues.
17. The method according to item 16, wherein the cysteine residues account for more than 4 % of the total amino acids of the human microbiota protein.
18. The method according to item 16 or 17, wherein the human microbiota protein contains an even number of cysteine residues, e.g. forming at least one or two cysteine pairs.
19. The method according to any one of items 16 to 18, wherein the cysteine-content of the human microbiota protein is identified using KAPPA.
20. The method according to any one of items 1 to 19, wherein the primary and/or a secondary structure element conferring a conformational rigid structure is selected from the group consisting of cysteine motif, leucine-rich repeat, alpha-helix, beta-sheet and coil.
21. The method according to any one of items 1 to 20, wherein the primary and/or a secondary structure element conferring a conformational rigid structure is a disulfide bridge.
22. The method according to any one of items 1 to 21, wherein the human microbiota protein has a length of 20 - 200 amino acids and comprises at least two cysteine residues, e.g. forming at least one cysteine pair.
23. The method according to any one of items 1 to 22, wherein the human microbiota protein has a length of 50 - 150 amino acids and comprises at least four cysteine residues, e.g. forming at least two cysteine pairs.
24. The method according to any one of items 1 to 22, wherein the human microbiota protein has a length of 20 - 50 amino acids and comprises a secondary structure element selected from the group consisting of an alpha-helix, a beta-sheet and a coil.
25. The method according to any one of items 1 to 24, wherein in step (ii) identification of sequences having or encoding a signal peptide is performed before:
   - identification of sequences having or encoding a sequence having a length of 20 - 500 amino acids; and/or
   - identification of sequences comprising at least two cysteine residues and/or a primary and/or a secondary structure element conferring a conformational rigid structure.
26. The method of item 25, wherein the sequence of the signal peptide is not included:
   - in the identification of sequences having or encoding a sequence having a length of 20 - 500 amino acids; and/or
   - in the identification of sequences comprising at least two cysteine residues and/or a primary and/or a secondary structure element conferring a conformational rigid structure.
27. The method according to any one of items 1 to 26, wherein in step (ii) protein or nucleic acid sequences with unknown functions are annotated, preferably using an *in silico* method.
28. The method according to item 27, wherein the sequences are annotated by using HMMSCAN and/or PFAM.
29. The method according to any one of items 1 - 28, wherein in step (ii) redundant sequences are identified and removed.
30. The method according to item 29, wherein in step (ii) sequences having at least 95%, preferably at least 90%, more preferably at least 85%, even more preferably at least 80% and most preferably at least 75% sequence identity to another sequence are identified and removed.
31. The method according to item 29 or 30, wherein redundant sequences are identified by using CD-HIT.
32. The method according to any one of items 1 - 31, wherein in step (ii) signal peptides of secreted proteins are distinguished from lipoproteins of gram-negative bacteria.
33. The method of item 32, wherein the step of distinguishing signal peptides of secreted proteins from lipoproteins of gram-negative bacteria is performed using LipoP.
34. The method according to any one items 1 to 33, wherein the human microbiota protein is a mimic or a secretagogue of a human host protein, e.g. selected from the group consisting of cytokines, interleukins, chemokines, growth factors, neuropeptides and peptide hormones.
35. The method according to any one items 1 to 34, wherein the human microbiota protein is a secretagogue.
36. The method according to item 35, wherein the secretagogue induces secretion of Interleukin-10 (IL-10) by human immune cells.
37. The method according to any one of items 1 - 36, wherein the identified protein is immunomodulatory.
38. The method according to any one of items 1 - 37, wherein the human microbiota protein is prepared in step (iii) by chemical synthesis.
39. The method according to any one of items 1 - 38, wherein the human microbiota protein is prepared in step (iii) by *in vitro* synthesis (cell-free expression) or by recombinant overexpression.
40. The method according to item 39, wherein a nucleic acid molecule is used as template for preparing the protein and wherein the method comprises the step of
   - Identifying a start and a stop codon in the nucleic acid sequence.
41. The method according to any one of items 1 to 40, wherein the human microbiota protein prepared in step (iii) is synthetized without the signal peptide identified in step (ii).
42. The method according to item 41, wherein the signal peptide is removed *in silico* before preparation of the protein.
43. The method according to any one of items 1 to 42, wherein the method comprises an additional step (iv) of determining at least one biological activity of the (obtained) protein(s), in particular relating to an interaction with the human host.
44. The method of item 43, wherein the biological activity is tested *in silico.*
45. The method according to item 43 or 44, wherein the structure of the human microbiota protein is determined and compared to the structure of a human host molecule, in particular a human host protein.
46. The method according to any one of items 43 to 45, wherein the biological activity is tested *in vitro* or *in vivo.*
47. The method according to item 46, wherein the biological activity test is selected from the group consisting of co-immunoprecipitation, bimolecular fluorescence complementation, affinity electrophoresis, label transfer, phage display, tandem affinity purification, photo-reactive amino acid analogues (in vivo), SPINE, quantitative immunoprecipitation combined with knock-down (QUICK), bio-layer interferometry, Dual polarisation interferometry (DPI), Static light scattering (SLS), Dynamic light scattering (DLS), Surface plasmon resonance, Fluorescence polarization/anisotropy, fluorescence correlation spectroscopy,fluorescence cross-correlation spectroscopy (FCCS), Fluorescence resonance energy transfer (FRET), NMR, Isothermal titration calorimetry (ITC), Microscale thermophoresis (MST), Rotating cell-based ligand binding assay, Single colour reflectometry (SCORE), microarrays in particular peptide arrays and protein arrays, and display methods such as peptide phage display, yeast surface display, yeast two-hybrid and bacterial two-hybrid screen.
48. The method according to any one of items 43 to 47, wherein the biological activity is tested by a screening method to investigate the involvement of the obtained protein in a biological mechanism, such as metabolism, immunity or cellular integrity.
49. The method according to any one of items 43 to 48, wherein the obtained protein is tested using an assay dedicated to G-protein coupled receptors (GPCRs).
50. The method according to any one of items 43 to 49, wherein the obtained protein is tested using an assay relating to the induction of calcium release from human cells.
51. The method according to any one of items 43 to 50, wherein the obtained protein is tested using an assay relating to immunity, optionally involving human immune cells, such as peripheral blood mononuclear cells (PBMCs) or a selected subset of human immune cells.
52. The method according to any one of items 43 to 51, wherein cytokine release from human cells exposed to the obtained protein is determined.
53. A library of human microbiota protein drug candidates obtainable by the method according to any one items 1 to 52.
54. Method for identification of a protein of the human microbiota metasecretome, the method comprising the following steps:
   (i) providing a plurality of human microbiota protein sequences and/or a plurality of nucleic acid sequences encoding a plurality of human microbiota proteins;
   (ii) identifying in the sequences provided in step (i) one or more sequence(s) of (a) protein(s) of the human microbiota metasecretome, wherein the sequence(s) of the protein of the human microbiota metasecretome is/are selected according to the following criteria:
      - (a) sequence(s) having, or coding for proteins having, a signal peptide;
      - (a) sequence(s) having, or coding for proteins having, a length of 20 - 500 amino acids; and
      - (a) sequence(s) comprising, or coding for (a) protein(s) comprising, at least two cysteine residues and/or a primary and/or a secondary structure element conferring a conformational rigid structure.
55. The method according to item 54, which is performed as defined in any one of items 2 - 37.
56. Method for generating a human microbiota metasecretome sequence database comprising the identification of a plurality of human microbiota metasecretome sequences according to item 54 or 55.
57. The method according to item 56, wherein in step (i) a plurality of human microbiota protein sequences and/or a plurality of nucleic acid sequences encoding a plurality of human microbiota proteins is provided and compiled in a database and wherein the subsequent steps are performed on the database or on the sequences contained in the database.
58. Method for preparing a protein of the human microbiota metasecretome comprising the identification of a plurality of human microbiota metasecretome protein sequences and/or of a plurality of nucleic acid sequences encoding a plurality of human microbiota proteins according to item 54 or 55 followed by a step of
   - preparing one or more protein(s) of the human microbiota metasecretome based on the previously identified sequence(s).
59. The method for preparing a protein of the human microbiota metasecretome, wherein the protein is prepared as defined in any one of items 38 to 42.
60. Method for identifying and/or providing a protein of the human microbiota metasecretome interacting with a human host molecule comprising
   - the identification of a plurality of human microbiota metasecretome protein sequences and/or of a plurality of nucleic acid sequences encoding a plurality of human microbiota proteins according to item 54 or 55,
   - and, optionally, the preparation of one or more protein(s) of the human microbiota metasecretome according to item 58 or 59,
   followed by a step of testing the interaction of the identified/prepared protein of the human microbiota metasecretome with a human host molecule, in particular a human host protein.
61. The method according to item 60, wherein the biological activity is tested as defined in any one of items 44 to 52.
62. A human microbiota metasecretome protein obtainable by the method according to any one of items 1 to 52 and 58 to 61 for use in medicine.
63. The human microbiota metasecretome protein for use according to item 62 comprising an amino acid sequence according to any one of SEQ ID NOs 1 - 10.
64. Method for preparing a medicament for prevention and/or treatment of a disease comprising the following steps:
   (a) providing a human microbiota protein drug candidate according to any one of items 1 to 52 and 54 to 61;
   (b) preparing a pharmaceutical composition comprising:
      (1) said protein of the human microbiota metasecretome or a functional fragment or sequence variant thereof;
      (2) a nucleic acid molecule encoding the protein according to (1);
      (3) a cell expressing the protein according to (1) or comprising the nucleic acid molecule according to (2);
      (4) an antibody binding to the protein according to (1);
      (5) a nucleic acid molecule encoding the antibody according to (4);
      (6) a cell expressing the antibody according to (4) or comprising the nucleic acid molecule according to (5);
      (7) a compound interacting with the protein according to (1); or
      (8) a compound interfering with the interaction (binding) of the protein according to (1) to the human host molecule
      and, optionally, a pharmaceutically acceptable carrier and/or an adjuvant.

The invention, and in particular the items outlined above, are described in more detail below.

### Method for providing a human microbiota protein drug candidate

In a first aspect the present invention provides a method for providing a human microbiota protein drug candidate, the method comprising the following steps:
(i) providing a plurality of human microbiota protein sequences and/or a plurality of nucleic acid sequences encoding a plurality of human microbiota proteins;
(ii) identifying in the sequences provided in step (i) one or more sequence(s) of (a) human microbiota protein drug candidate(s), wherein the sequence(s) of the human microbiota protein drug candidate(s) is/are selected according to the following criteria:
   - (a) sequence(s) having, or coding for proteins having, a signal peptide;
   - (a) sequence(s) having, or coding for proteins having, a length of 20 - 500 amino acids; and
   - (a) sequence(s) comprising, or coding for (a) protein(s) comprising, at least two cysteine residues and/or a primary and/or a secondary structure element conferring a conformational rigid structure; and
(iii) preparing one or more human microbiota protein(s) having, or encoded by, the sequence(s) identified in step (ii).

Without being bound to any theory, the present inventors assume that commensal microbiota, in particular microbiota of the human microbiome, have optimized their secreted proteins for interaction/communication with their environment, including in particular their (human) host but also the microbiota communities such as the bacteria-bacteria interactions, by virtue of several thousand generations of bacterial evolution, in particular regarding activity, bioavailability, toxicity etc. In view thereof, proteins of the human microbiome metasecretome represent ideal drug candidates for the prevention and treatment of various (human) diseases. Therefore, in order to identify proteins interacting with the human host among the many proteins expressed by the microbiota residing in the human host, the present inventors focused on the metasecretome of human microbiota. It is assumed that the crosstalk between human microbiota and the human host is mediated by direct cell-cell contact or by small molecules secreted by cells. Accordingly, microbiota proteins located at the cell surface or being secreted are involved interactions, such as the microbiota-host interactions.

In view thereof, the "human microbiota protein drug candidate" is a microbial protein expressed (or predicted to be expressed) by microbiota of the human microbiome, namely, of the metasecretome of the human microbiome. Therefore, it is also often referred to the "human microbiota protein drug candidate" herein by "human microbiota (metasecretome) protein" or "protein of the human microbiota (metasecretome)" and the like.

In the context of the present invention, i.e. throughout the present application, the terms "peptide", "polypeptide", "protein" and variations of these terms refer to peptides, oligopeptides, polypeptides, or proteins comprising at least two amino acids joined to each other preferably by a normal peptide bond, or, alternatively, by a modified peptide bond, such as for example in the cases of isosteric peptides. In particular, the term "protein" refers to peptides and proteins, independent of their length. Accordingly, the term "protein" in particular includes short peptides (for example oligopeptides) as well as long(er) polypeptides and proteins.

The terms "peptide", "polypeptide", "protein" may also include "peptidomimetics", which are defined as peptide analogs containing non-peptidic structural elements, which peptides are capable of mimicking or antagonizing the biological action(s) of a natural parent peptide. A peptidomimetic lacks classical peptide characteristics such as enzymatically scissile peptide bonds. In particular, a peptide, polypeptide or protein can comprise amino acids other than the 20 amino acids defined by the genetic code in addition to these amino acids, or it can be composed of amino acids other than the 20 amino acids defined by the genetic code. In particular, a peptide, polypeptide or protein in the context of the present invention can equally be composed of amino acids modified by natural processes, such as post-translational maturation processes or by chemical processes, which are well known to a person skilled in the art. Such modifications are fully detailed in the literature. These modifications can appear anywhere in the polypeptide: in the peptide skeleton, in the amino acid chain or even at the carboxy- or amino-terminal ends. In particular, a peptide or polypeptide can be branched following an ubiquitination or be cyclic with or without branching. This type of modification can be the result of natural or synthetic post-translational processes that are well known to a person skilled in the art. The terms "peptide", "polypeptide", "protein" in the context of the present invention in particular also include modified peptides, polypeptides and proteins. For example, peptide, polypeptide or protein modifications can include acetylation, acylation, ADP-ribosylation, amidation, covalent fixation of a nucleotide or of a nucleotide derivative, covalent fixation of a lipid or of a lipidic derivative, the covalent fixation of a phosphatidylinositol, covalent or non-covalent crosslinking, cyclization, disulfide bond formation, demethylation, glycosylation including pegylation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature (Proteins Structure and Molecular Properties (1993) 2nd Ed., T. E. Creighton, New York ; Post-translational Covalent Modifications of Proteins (1983) B. C. Johnson, Ed., Academic Press, New York ; Seifter et al. (1990) Analysis for protein modifications and nonprotein cofactors, Meth. Enzymol. 182: 626-646 and Rattan et al., (1992) Protein Synthesis: Post-translational Modifications and Aging, Ann NY Acad Sci, 663: 48-62). Accordingly, the terms "peptide", "polypeptide", "protein" preferably include for example lipopeptides, lipoproteins, glycopeptides, glycoproteins and the like.

In some embodiments, the protein of the human microbiota metasecretome according to the present invention is a "classical" (poly)peptide or protein, whereby a "classical" (poly)peptide/protein is typically composed of amino acids selected from the 20 amino acids defined by the genetic code, linked to each other by a normal peptide bond.

The term "microbiota", as used herein, refers to commensal, symbiotic and pathogenic microorganisms found in and on all multicellular organisms studied to date from plants to animals. In particular, microbiota have been found to be crucial for immunologic, hormonal and metabolic homeostasis of their host. Microbiota include bacteria, archaea, protists, fungi, viruses and phages. Accordingly, the human microbiota protein may be a bacterial protein, an archaea protein, a protist protein, a fungi protein, a virus protein and/or a phage protein. Preferably, the human microbiota protein is a bacterial protein or an archaea protein. More preferably, the microbiota protein is a bacterial protein.

Anatomically, microbiota reside on or within any of a number of tissues and biofluids, including the gastrointestinal tract, in particular the gut (and the oral cavity, in particular the oral mucosa), skin, conjunctiva, mammary glands, vagina, placenta, seminal fluid, uterus, ovarian follicles, lung and saliva. Accordingly, the microbiota may be selected from the group consisting of gastrointestinal tract microbiota, lung microbiota, saliva microbiota, seminal fluid microbiota, skin microbiota and vagina microbiota. In some embodiments, the microbiota are gastrointestinal tract microbiota selected from gut microbiota and oral cavity microbiota. Accordingly, the protein of the human microbiota metasecretome may be a microbial protein, which is expressed by microbiota residing in the human gastrointestinal tract, i.e. by "human gastrointestinal tract microbiota". This includes, for example, proteins expressed by microbiota residing in the gut and/or in the oral cavity of a human. In other embodiments, the protein of the human microbiota protein is a microbial protein, which is expressed by microbiota residing in tissues and body fluids other than (outside the) gastrointestinal tract, e.g. in the skin or the genital system (in particular the vagina). Preferably, the protein is a protein of the human gastrointestinal tract microbiota metasecretome, such as the human gut microbiota metasecretome, i.e. a microbial protein, which is expressed by microbiota residing in the human gastrointestinal tract, in particular in the human gut. More preferably, the protein is a protein of the human gut bacteria metasecretome, i.e. a bacterial protein, which is expressed by bacteria residing in the human gut.

The present invention relates in particular to microbiota found in and on humans. Such microbiota are also referred to herein as "human microbiota" (wherein the term human refers specifically to the localization/residence of the microbiota).

As used herein, the term "metasecretome" refers to a collection of secreted proteins, outer surface proteins, inner surface proteins, and transmembrane proteins from environmental (microbial) communities (microbiome), such as the microbial communities residing on or within human tissues and/or biofluids as described above, for example in the human gastrointestinal tract. Accordingly, the expression "human microbiota metasecretome" refers to a collection of secreted proteins, outer surface proteins and transmembrane proteins from microbiota residing in (or on) human hosts. Without being bound to any theory it is assumed that the metasecretome open reading frames (ORFs) comprises only 10% to 30% of total metagenome. The term "secretome" refers to a collection of proteins consisting of transmembrane proteins (TM), outer surface proteins, inner surface proteins, and proteins secreted by cells into the extracellular milieu/space. In some embodiments, the metasecretome protein is a secreted protein. In other words, in some embodiments, the protein is released from the microbiota (expressing the protein), in particular into the extracellular milieu/space, and not attached to said microbiota (or its outer surface).

In general, in the context of the present invention a protein of the human microbiota metasecretome may be identified based on its protein sequence (amino acid sequence) or based on a nucleic acid sequence encoding its protein sequence. Accordingly, every step of the method according to the present invention may be performed on protein sequences (amino acid sequences) and/or on nucleic acid sequences encoding said protein sequences. Accordingly, the term "sequence/sequences" as used herein (without any further specification) refers to protein sequences (amino acid sequences) and/or on nucleic acid sequences (encoding said protein sequences).

In general, the term "nucleic acid" or "nucleic acid molecule" includes any nucleic acid, such as single stranded, double stranded or partially double stranded nucleic acids, preferably selected from genomic DNA, cDNA, RNA, siRNA, antisense DNA, antisense RNA, ribozyme, complementary RNA/DNA sequences with or without expression elements, a mini-gene, gene fragments, regulatory elements, promoters, and combinations thereof. Further examples of nucleic acid (molecules) and/or polynucleotides include, e.g., a recombinant polynucleotide, a vector, an oligonucleotide, an RNA molecule, such as an mRNA, or a DNA molecule as described above. Accordingly, the nucleic acid (molecule) may be a DNA molecule or an RNA molecule; preferably selected from genomic DNA; cDNA; mRNA; RNA and/or DNA sequences with or without expression elements, regulatory elements, and/or promoters; a vector; and combinations thereof.

### Providing a plurality of human microbiota protein sequences

In step (i) of the method for identification of a protein of the human microbiota according to the present invention a plurality of human microbiota protein sequences and/or a plurality of nucleic acid sequences encoding a plurality of human microbiota proteins is provided.

It is understood that the term "human microbiota protein sequence" includes predicted protein sequences. Accordingly, a "nucleic acid sequence" encoding a human microbiota protein may be a nucleic acid sequence encoding a predicted microbiota protein, or a nucleic acid sequence predicted as encoding a microbiota protein.

In the field of the (human) microbiome, identification of microbiome proteins is widely performed by prediction of encoded proteins based on available nucleic acid sequences, which are usually obtained with modern high-throughput sequencing, such as shotgun next generation sequencing methods. For example, with regard to the gut microbiome, fecal sample microbiota are often analyzed as a proxy for gut microbiota employing whole genome shotgun next-generation sequencing (NGS). Thereby, sequences of short nucleic acid segments ("reads") are obtained, and multiple overlapping reads can be assembled into a continuous sequence ("contig"). Microbiome contigs may be obtained by methods known in the art or downloaded from public libraries, for example "HMASM2 - Assembled Metagenomes" from the NIH Human Microbiome Project (URL: https://www.hmpdacc.org/hmasm2/). In order to identify microbiome genes and proteins in such sequences, prokaryotic gene-finding tools may be used. Non-limiting examples of prokaryotic gene-finding tools include:
- tools of the GeneMark-family (URL: http://exon.gatech.edu/GeneMark/), such as GeneMarkS (URL: http://exon.gatech.edu/GeneMark/genemarks.cgi; John Besemer, Alexandre Lomsadze and Mark Borodovsky, GeneMarkS: a self-training method for prediction of gene starts in microbial genomes. Implications for finding sequence motifs in regulatory regions. Nucleic Acids Research (2001) 29, pp 2607-2618); GeneMark.hmm prokaryotic (URL: http://exon.gatech.edu/GeneMark/gmhmmp.cgi; Alexander Lukashin and Mark Borodovsky, GeneMark.hmm: new solutions for gene finding. Nucleic Acids Research (1998) 26, pp 1107-1115); MetaGeneMark (URL: http://exon.gatech.edu/GeneMark/meta_gmhmmp.cgi; Wenhan Zhu, Alex Lomsadze and Mark Borodovsky, Ab initio gene identification in metagenomic sequences Nucleic Acids Research (2010) 38, e132); and GeneMarkS2 (URL: http://exon.gatech.edu/GeneMark/genemarks2.cgi; Lomsadze A, Gemayel K, Tang S, Borodovsky M, Modeling leaderless transcription and atypical genes results in more accurate gene prediction in prokaryotes. Genome Res, 2018, 29(7), pp 1079-1089);
- GLIMMER (Gene Locator and Interpolated Markov ModelER) with GLIMMER 3.0 being currently the latest version (URL: http://ccb.jhu.edu/software/glimmer/index.shtml; S. Salzberg, A. Delcher, S. Kasif, and O. White. Microbial gene identification using interpolated Markov models, Nucleic Acids Research 26:2 (1998), 544-548; A.L. Delcher, K.A. Bratke, E.C. Powers, and S.L. Salzberg. Identifying bacterial genes and endosymbiont DNA with Glimmer, Bioinformatics 23:6 (2007), 673-679); and
- PRODIGAL (Prokaryotic Dynamic Programming Genefinding Algorithm) and MetaProdigal (URL: http://code.google.com/p/prodigal/; Hyatt D, Chen GL, Locascio PF, Land ML, Larimer FW, Hauser LJ, Prodigal: prokaryotic gene recognition and translation initiation site identification. BMC Bioinformatics11 (1):119 (2010); Hyatt D, LoCascio PF, Hauser LJ, Uberbacher EC. Gene and translation initiation site prediction in metagenomic sequences. Bioinformatics. 2012;28(17):2223-30).

Among those gene-finding tools, the GeneMark-family, in particular GeneMarkS2, is preferred.

The plurality of human microbiota protein sequences and/or the plurality of nucleic acid sequences encoding a plurality of human microbiota proteins is preferably provided on basis of a database. Accordingly, in step (i) preferably a database of human microbiota protein sequences and/or a database of nucleic acid sequences encoding, or predicted as encoding, human microbiota proteins is provided. The term "database" refers to an (organized) collection of data. Accordingly, any collection of human microbiota protein or nucleic acid sequence data may serve as starting point to provide a plurality of human microbiota protein sequences and/or a plurality of nucleic acid sequences encoding a plurality of human microbiota proteins.

As used herein, the term "plurality" typically refers to more than one, for example 2, 3, 4, 5, 6, 7, 8, 9, 10, 11, 12, 13, 14, 15, 16, 17, 18, 19, 20, or more, e.g., 25, 30, 35, 40, 45, 50, 60, 70, 80, 90, 100 or more items (sequences). In particular, the items of a plurality are distinct. Accordingly, the expression "a plurality of human microbiota proteins/protein sequences" refers to at least two distinct human microbiota proteins/protein sequences and the expression "a plurality of nucleic acid sequences" refers to at least two distinct nucleic acid sequences.

Preferably, the plurality of human microbiota protein sequences and/or nucleic acid sequences are provided in step (i) on basis of a (microbiota) (sequence) database. Such databases preferably comprise human microbiota sequences of multiple human individuals (host individuals; human subjects).

A preferred example of such a database is the "Integrated reference catalog of the human gut microbiome" (version 1.0, March 2014; Li et al., MetaHIT Consortium. An integrated catalog of reference genes in the human gut microbiome. Nat Biotechnol. 2014 Aug;32(8):834-41; URL: http://meta.genomics.cn/meta/home), which includes data from the major human microbiome profiling efforts, the American National Institutes of Health Human Microbiome Project (NIH-HMP) and the European Metagenomics of the Human Intestinal Tract Initiative (MetaHIT).

Another preferred example of such a database is the Unified Human Gastrointestinal Genome (UHGG) collection, a resource combining 286,997 genomes representing 4,644 prokaryotic species from the human gut (Alexandre Almeida, Stephen Nayfach, Miguel Boland, Francesco Strozzi, Martin Beracochea, Zhou Jason Shi, Katherine S. Pollard, Donovan H. Parks, Philip Hugenholtz, Nicola Segata, Nikos C. Kyrpides, Robert D. Finn. A unified sequence catalogue of over 280,000 genomes obtained from the human gut microbiome. bioRxiv 762682; 19 Sep 2019, doi: https://doi.org/10.1101/762682). These genomes contain over 625 million protein sequences used to generate the Unified Human Gastrointestinal Protein (UHGP) catalogue, a collection that more than doubles the number of gut protein clusters over the Integrated Gene Catalogue.

Moreover, another preferred example is a database, which is compiled by selecting human microbiota sequences from a larger database. For example, microbiota species known to reside in the human intestinal tract may be selected (e.g., based on literature known to the skilled person) and (all available) sequences of those selected microbiota species may be retrieved from larger microbiota databases, for example from the Ensembl Bacteria database (URL: http://bacteria.ensembl.org/index.html; P.J. Kersey, J.E. Allen, A. Allot, M. Barba, S. Boddu, B.J. Bolt, D. Carvalho-Silva, M. Christensen, P. Davis, C. Grabmueller, N. Kumar, Z. Liu, T. Maurel, B. Moore, M. D. McDowall, U. Maheswari, G. Naamati, V. Newman, C.K. Ong, D.M. Bolser., N. De Silva, K.L. Howe, N. Langridge, G. Maslen, D.M. Staines, A. Yates. Ensembl Genomes 2018: an integrated omics infrastructure for non-vertebrate species Nucleic Acids Research 2018 46(D1) D802-D808).

In some embodiments, the database may be compiled in relation to a selected disease or disorder, for example to identify microbial proteins interacting with the human host for treatment of the selected disorder. In this context, the database may focus on sequences of human microbial species, e.g. of the gastrointestinal tract, associated with said disorder. Moreover, such a database may contain sequences identified, for example, in samples, such as stool samples, of patients diagnosed with the selected disease/disorder and/or of subject resistant/immune to the selected disease/disorder.

Accordingly, a database may be also compiled by the sequencing of (stool) samples containing human microbiota.

In some embodiments, various databases, for example as described above, may be pooled to obtain a database containing as many human microbiota sequences as possible.

In some instances, the database may comprise human microbiota sequences of a single human individual (host individual), but not of multiple human individuals (host individuals). Such databases may be advantageous, for example, in a personalized medicine approach. A database comprising microbiota data of a single individual, but not of multiple individuals, may be compiled, for example, by the use of one or more stool samples of the individual. For example, microbial (in particular bacterial) nucleic acids (such as DNA) or (poly)peptides may be extracted from the stool sample and sequenced by methods known in the art. The sequences may then be compiled in a database containing only microbiota data, in particular sequences. For example, the sequencing of the DNA extracted from a stool sample may be performed, e.g. at 40 million pair end reads for example on an Illumina HiSeq. Sequences can be analyzed, for example, using bioinformatics pipeline for identification of genomic part of candidate bacteria expressing the protein of the human microbiota metasecretome (e.g., a bacterial peptide).

Step (i) of the method for providing a human microbiota protein drug candidate according to the present invention may optionally comprise the following sub-steps:
(i-a) optionally, identifying microbiota protein sequences or nucleic acid sequences from (a) sample(s) of a single or multiple individual(s), and
(i-b) compiling a database containing microbiota protein sequences or nucleic acid sequences of a single or multiple individual(s).

The sample in step (i-a) may be a stool sample. Depending on whether the database to be compiled shall relate to a single or multiple individual(s), one or more stool samples of a single or multiple individual(s) may be used. The identification step (i-a) preferably comprises extraction of microbial (in particular bacterial) nucleic acids (such as DNA) or (poly)peptides from the sample, in particular the stool sample and sequencing thereof, e.g. as described above. Optionally, sequences may be analyzed as described above.

### Identification of a human microbiota protein drug candidate

In step (ii) of the method for providing a human microbiota protein drug candidate according to the present invention, a drug candidate is identified among the microbiota sequences provided in step (i). This drug candidate is a human microbiota protein (i.e., a microbial protein expressed or predicted to be expressed by the human microbiome), which fulfills three important criteria:
- it contains (in its uncleaved (prodrug) version) a signal peptide;
- it has a length of 20 - 500 amino acids; and
- it comprises (a) at least two cysteine residues, and/or (b) a primary and/or secondary structure element conferring a conformational rigid structure.
Accordingly, such protein sequences, or nucleic acid sequences (predicted to) encoding such protein sequences, are identified and selected in step (ii) among the sequences provided in step (i).

The present inventors identified the above three criteria for potential microbiota drug candidates, because they indicate that the microbiota protein is involved in interactions with the host, as reflected by the signal peptide and the relatively small size, which corresponds to the size of known interacting molecules, such as cytokines (e.g. interleukins), chemokines, growth factors, neuropeptides, peptide hormones etc. Moreover, cysteines or other structure elements conferring a conformational rigid structure "constrain" the microbiota protein in the proper conformation, such that it can bind to appropriate (human) targets, such as receptors or other interacting proteins. Therefore, human microbiota proteins fulfilling the above-mentioned three criteria represent - with the highest probability - protein drug candidates that can then be prepared and, optionally, tested for their biological effect by implementing relevant *in vitro* or *in vivo* assays as explained below.

The sequences obtained in step (i) may be tested for the three selection criteria in step (ii) in any order; for example
- first testing for a signal peptide, thereafter the length and then the cysteines or other element conferring a rigid structure,
- first testing for a signal peptide, thereafter the cysteines or other element conferring a rigid structure and then the length,
- first testing the length, thereafter for a signal peptide and then the cysteines or other element conferring a rigid structure,
- first testing the length, thereafter the cysteines or other element conferring a rigid structure and then for a signal peptide,
- first testing for the cysteines or other element conferring a rigid structure, thereafter the length and then for a signal peptide, or
- first testing for the cysteines or other element conferring a rigid structure, thereafter for a signal peptide and then for the length.

Preferably, among the sequences provided in step (i) such sequences comprising (or encoding/predicted to encode) a signal peptide are identified before the length and/or the cysteine content (or a structure element conferring a conformational rigid structure) of the (encoded) protein is determined. The sequence of the signal peptide may then be removed ("cleaved", e.g. *in silico*), e.g. before the length and/or the cysteine content (or a structure element conferring a conformational rigid structure) of the (encoded) protein is determined. The length or cysteine content (or other structure elements) of the signal peptide are usually not relevant for the host interaction of the "mature" microbiota protein, because signal peptides are often cleaved after transport/"release" of the protein (e.g. outside the microbiota cell). Therefore, in step (ii) preferably the presence of a signal peptide is determined before the length and/or the cysteine content (or a structure element conferring a conformational rigid structure) of the (encoded) protein is determined. Sequences not containing a signal peptide may be "discarded", i.e. not considered for further analysis. In some embodiments, the signal peptide is removed ("cleaved", e.g. *in silico*) from the selected sequences (previously) containing (or coding for) a signal peptide, such that the signal peptide is not considered in the further analysis.

Accordingly, it is preferred that in step (ii) identification of sequences having or encoding a signal peptide is performed before:
- identification of sequences having or encoding a sequence having a length of 20 - 500 amino acids; and/or
- identification of cysteine residues or sequences comprising a primary and/or a secondary structure element conferring a conformational rigid structure.

Moreover, it is preferred that the sequence of the signal peptide is not included:
- in the identification of sequences having or encoding a sequence having a length of 20 - 500 amino acids; and/or
- in the identification of cysteine residues or sequences comprising a primary and/or a secondary structure element conferring a conformational rigid structure.

In some embodiments, the cleavage site given by the Y score from SignalP may be used to remove the signal peptide. In some cases, the two modes of prediction for SignalP, the gram-positive and gram-negative, can both predict a positive signal peptide on a protein but with a different cleavage position. In such cases, the smaller cleavage site may be considered, which is defined as the smallest coordinate in the protein sequence between the two SignalP predictions, where the software has indicated the end of the signal peptide.

Moreover, further preferred examples of bioinformatics tools (software) to identify the signal peptide cleavage site include:
- EMBOSS SigCleave (URL: http://emboss.bioinformatics.nl/cgi-bin/emboss/sigcleave; von Heijne, G. "A new method for predicting signal sequence cleavage sites" Nucleic Acids Res.: 14:4683 (1986); von Heijne, G. "Sequence Analysis in Molecular Biology: Treasure Trove or Trivial Pursuit" (Acad. Press, (1987), 113-117)); Peter M. Rice,Peter M. Rice, Alan J. Bleasby, Jon C. Ison,Alan J. Bleasby,Jon C. Ison: EMBOSS User's Guide: Practical Bioinformatics, Cambridge University Press, 1st edition, June 2011);
- SignalCF (URL: http://www.csbio.sjtu.edu.cn/bioinf/Signal-CF/; Kuo-Chen Chou and Hong-Bin Shen, Signal-CF: A subsite-coupled and window-fusing approach for predicting signal peptides, Biochem Biophys Res Comm, 2007,357: 633-640);
- SPEPlip (URL: http://gpcr.biocomp.unibo.it/cgi/predictors/spep/pred_spepcgi.cgi; Piero Fariselli, Giacomo Finocchiaro, Rita Casadio; SPEPlip: the detection of signal peptide and lipoprotein cleavage sites, Bioinformatics, Volume 19, Issue 18, 12 December 2003, Pages 2498-2499);
- PrediSi (URL: http://www.predisi.de/; Hiller K, Grote A, Scheer M, Münch R, Jahn D. PrediSi: prediction of signal peptides and their cleavage positions. Nucleic Acids Res. 2004 Jul 1;32(Web Server issue):W375-9)); and
- ANTHEPROT (URL: http://antheprot-pbil.ibcp.fr/signal_prediction.html; Deleage G, Combet C, Blanchet C, Geourjon C. ANTHEPROT: an integrated protein sequence analysis software with client/server capabilities. Computers in biology and medicine. 2001;31 :259-267).

In some embodiments, it may be determined whether the length of the (encoded) microbiota protein is 20 - 500 amino acids before the cysteine-content (or a structure element conferring a conformational rigid structure) of the (encoded) protein is determined. For example, the cysteine content may be expressed as percentage (cysteines : total amino acids). In those cases, the length of the protein is required in order to calculate the cysteine percentage.

In view of the above, it may be advantageous to test the three criteria in the following order:
1. selecting proteins containing (in its uncleaved (preprotein/prodrug) version) a signal peptide;
2. selecting proteins having a length of 20 - 500 amino acids; and
3. selecting proteins comprising at least two cysteine residues and/or a primary and/or secondary structure element conferring a conformational rigid structure.

Sequences of microbiota proteins (or nucleic acid sequences encoding microbiota proteins), which do not fulfill all three of the above-mentioned criteria are disregarded in the further analysis ("discarded"). In other words, only such sequences of microbiota proteins (or nucleic acid sequences encoding microbiota proteins) are selected for the further steps of the method, which fulfill all three criteria and, thus, represent human microbiota protein drug candidates.

### Signal peptide

In step (ii), a plurality of sequences having a signal peptide is identified. Sequences having no signal peptide may be removed/deleted.

As used herein, the term "signal peptide" refers to a ubiquitous protein-sorting signal that targets its cargo protein for translocation across the cytoplasmic membrane. Typically, a signal peptide is a short peptide (e.g., 16-30 amino acids long), which is usually located at the N-terminus of a newly synthesized, to-be-secreted protein. Accordingly, presence of a signal peptide indicates that a protein is secreted. The term "signal peptide" may also include transmembrane-domains that serve as signal peptides, which are sometimes referred to as "signal anchor sequences".

In prokaryotes, signal peptides direct the newly synthesized protein to the SecYEG protein-conducting channel, which is present in the plasma membrane. The Sec pathway is a canonical pathway found in both gram-positive and gram-negative bacteria and it is typically used for the transport of unfolded proteins. Another protein targeting pathway is the Tat pathway, which is only used for a small fraction of proteins (e.g. 6% of secreted proteins in *E coli*). The Tat pathway is typically used for the transport of longer and already folded proteins, normally delivered in the periplasm. The Tat pathway is also based on a very similar signal peptide at the N-terminus of the protein, but with an additional two Arginine motif.

Signal peptides may be recognized by common structural motifs. The common structure of signal peptides from various proteins is usually characterized by a hydrophobic core containing a long stretch of hydrophobic amino acids (about 5-16 residues long) that has a tendency to form a single alpha-helix and is also referred to as the "h-region". Many signal peptides begin with a short positively charged stretch of amino acids, which may help to enforce proper topology of the polypeptide during translocation by what is known as the positive-inside rule. Because of its close location to the N-terminus it is called the "n-region". In addition, many signal peptides contain a neutral but polar "c-region" at their C-terminus. Signal peptides are usually cleaved at the end of the transportation process. The (-3,-1)-rule states that the residues at positions -3 and -1 (relative to the cleavage site) must be small and neutral for cleavage to occur correctly. However, this cleavage site is usually absent from transmembrane-domains that serve as signal peptides. Signal peptidase may cleave either during or after completion of translocation to generate a free signal peptide and a mature protein.

Moreover, a large number of signal peptide sequences is known in the art and available, for example, in signal peptide databases. Examples of such signal peptide databases include signal peptide website (URL: http://www.signalpeptide.de/), SPdb (URL: http://proline.bic.nus.edu.sg/spdb/; Choo KH, Tan TW, Ranganathan S. SPdb--a signal peptide database. BMC Bioinformatics. 2005 Oct 13;6:249), and LocSigDB (URL: http://genome.unmc.edu/LocSigDB/; Simarjeet Negi, Sanjit Pandey, Satish M. Srinivasan, Akram Mohammed, Chittibabu Guda; LocSigDB: a database of protein localization signals, Database, Volume 2015, 1 January 2015, bav003). In addition, signal peptides may be retrieved from annotations in protein databases, for example in UniProt (URL: https://www.uniprot.org/; The UniProt Consortium. UniProt: the universal protein knowledgebase. Nucleic Acids Res. 45: D158-D169 (2017)).

Moreover, a signal peptide may be determined based on bioassays. For example, a protein may be expressed and its location (e.g. inside or outside a cell, in or on the membrane) may be studied. To this end, the protein may be labelled or tagged. Furthermore, mutation analyses may be performed.

Preferably, however, the identification of a signal peptide is performed *in silico.* In some embodiments, a signal peptide may be identified by using a bioinformatic tool, which is able to discriminate between the highly similar hydrophobic region of a transmembrane helix and that of a signal peptide. Moreover, a bioinformatic tool may be used, which is using Hidden Markov Models or Neural Network based software.

Examples of preferred bioinformatics tools (software) to identify a signal peptide include:
- Phobius (A combined transmembrane topology and signal peptide predictor, Stockholm Bioinformatics Centre; URL: http://phobius.sbc.su.se/; Lukas Käll, Anders Krogh and Erik L. L. Sonnhammer. A Combined Transmembrane Topology and Signal Peptide Prediction Method. Journal of Molecular Biology, 338(5):1027-1036, May 2004; Lukas Käll, Anders Krogh and Erik L. L. Sonnhammer. Advantages of combined transmembrane topology and signal peptide prediction--the Phobius web server Nucleic Acids Res., 35:W429-32, July 2007);
- SignalP (current version: 4.1; Center for biological sequence analysis, Technical University of Denmark DTU; URL: www.cbs.dtu.dk/services/SignalP; Henrik Nielsen, Jacob Engelbrecht, Søren Brunak and Gunnar von Heijne. Identification of prokaryotic and eukaryotic signal peptides and prediction of their cleavage sites. Protein Engineering, 10:1-6, 1997; Thomas Nordahl Petersen, Søren Brunak, Gunnar von Heijne & Henrik Nielsen. SignalP 4.0: discriminating signal peptides from transmembrane regions. Nature Methods, 8:785-786, 2011):
- PSORT (URL: https://psort.hgc.jp/; Nakai, K. and Horton, P., PSORT: a program for detecting the sorting signals of proteins and predicting their subcellular localization, Trends Biochem. Sci, 24(1) 34-35 (1999));
- SignalCF (URL: http://www.csbio.sjtu.edu.cn/bioinf/Signal-CF/; Kuo-Chen Chou and Hong-Bin Shen, Signal-CF: A subsite-coupled and window-fusing approach for predicting signal peptides, Biochem Biophys Res Comm, 2007,357: 633-640);
- Signal-3L (URL: http://www.csbio.sjtu.edu.cn/bioinf/Signal-3L/; Yi-Ze Zhang and Hong-Bin Shen, "Signal-3L 2.0: A hierarchical mixture model for enhancing protein signal peptide prediction by incorporating residue-domain cross level features", Journal of Chemical Information and Modeling, 2017, 57: 988-999; Hong-Bin Shen and Kuo-Chen Chou, "Signal-3L: a 3-layer approach for predicting signal peptides", Biochemical and Biophysical Research Communications, 2007, 363: 297-303);
- Signal-BLAST (URL: http://sigpep.services.came.sbg.ac.at/signalblast.html; Karl Frank; Manfred J. Sippl: High Performance Signal Peptide Prediction Based on Sequence Alignment Techniques. Bioinformatics, 24, pp. 2172-2176 (2008);
- PrediSi (URL: http://www.predisi.de/; Hiller K, Grote A, Scheer M, Münch R, Jahn D. PrediSi: prediction of signal peptides and their cleavage positions. Nucleic Acids Res. 2004 Jul 1;32(Web Server issue):W375-9));
- OCTOPUS/SPOCTOPUS (URL: http://octopus.cbr.su.se/; Viklund H, Elofsson A. OCTOPUS: improving topology prediction by two-track ANN-based preference scores and an extended topological grammar. Bioinformatics. 2008 Aug 1;24(15):1662-8; Viklund H, Bernsel A, Skwark M, Elofsson A. SPOCTOPUS: a combined predictor of signal peptides and membrane protein topology. Bioinformatics. 2008 Dec 15;24(24):2928-9);
- Philius (URL: http://www.yeastrc.org/philius; Reynolds SM, Käll L, Riffle ME, Bilmes JA, Noble WS (2008) Transmembrane Topology and Signal Peptide Prediction Using Dynamic Bayesian Networks. PLoS Comput Biol 4(11): e1000213);
- ANTHEPROT (URL: http://antheprot-pbil.ibcp.fr/signal_prediction.html; Deleage G, Combet C, Blanchet C, Geourjon C. ANTHEPROT: an integrated protein sequence analysis software with client/server capabilities. Computers in biology and medicine. 2001;31 :259-267);
- SOSUIsignal (URL: http://harrier.nagahama-i-bio.ac.jp/sosui/sosuisignal/sosuisignal_submit.html; Gomi M., Sonoyama M., and Mitaku S., High performance system for signal peptide prediction: SOSUIsignal. Chem-Bio Info. J., 4 142-147 (2004));
- TMHMM (URL: http://www.cbs.dtu.dk/services/TMHMM/; Krogh A, Larsson B, von Heijne G, Sonnhammer EL. Predicting transmembrane protein topology with a hidden Markov model: application to complete genomes. J Mol Biol. 2001;305(3):567-80; for prediction of transmembrane helices in proteins); and
- SPEPlip (URL: http://gpcr.biocomp.unibo.it/cgi/predictors/spep/pred_spepcgi.cgi; Piero Fariselli, Giacomo Finocchiaro, Rita Casadio; SPEPlip: the detection of signal peptide and lipoprotein cleavage sites, Bioinformatics, Volume 19, Issue 18, 12 December 2003, Pages 2498-2499).

One or more of these exemplified bioinformatics tools (software) may be used for the identification of a signal peptide in the method for providing a human microbiota protein drug candidate according to the present invention.

Preferably, the identification of a signal peptide in the method for providing a human microbiota protein drug candidate according to the present invention is performed by using a software for prediction of signal peptides, such as Phobius and/or SignalP. Such a software for prediction of signal peptides may be used by applying default settings. For example, the identification of a signal peptide in step (ii) is performed by using Phobius and/or SignalP.

In some embodiments, the method of the invention includes a single step of identification of a signal peptide, wherein a single method for identifying a signal peptide (e.g., a single bioinformatics tool as described above) is preformed once. More preferably, however, the identification of a signal peptide in step (ii) is performed by using two distinct methods, wherein a sequence having, or coding for, a signal peptide according to both methods is selected. In some embodiments, two distinct bioinformatic tools (i.e., two distinct methods) are used for the identification of the signal peptide, such that the obtained proteins with signal peptides correspond to those proteins identified by both methods. Thereby, the annotation confidence is improved. For example, Phobius and SignalP may be used (in combination) to identify proteins with signal peptides. In some embodiments, Phobius is used to identify proteins with signal peptides and, thereafter, SignalP is used to identify proteins with signal peptides, such that the obtained proteins with signal peptides correspond to those proteins identified by both methods. In other embodiments, SignalP is used to identify proteins with signal peptides and, thereafter, Phobius is used to identify proteins with signal peptides, such that the obtained proteins with signal peptides correspond to those proteins identified by both methods. Accordingly, the method of the invention may include more than one, e.g. two or three, steps of identification of a signal peptide.

While the more than one, e.g. two or three, steps of identification of a signal peptide may be performed directly after each other, it is also possible to perform other steps in between the two or more steps of identification of a signal peptide. For example, signal peptides may be determined by a first method, thereafter, other steps, e.g. annotating of the sequences and/or removing redundant sequences may be performed and, thereafter, the selected peptides may be checked for a signal peptide by a second method, which differs from the first method for identifying a signal peptide.

It is also preferred that a Tat signal peptide is identified (for example, in addition to the Sec signal peptide, which may be identified as described above). The Tat signal peptide can be detected with the same prediction methods as used for the Sec targeting pathway (i.e., the prediction methods as described above). It can also be identified by specialized bioinformatic tools/software, such as TatP (URL: http://www.cbs.dtu.dk/services/TatP/; Jannick Dyrløv Bendtsen, Henrik Nielsen, David Widdick, Tracy Palmer and Søren Brunak. Prediction of twin-arginine signal peptides. BMC bioinformatics 2005 6: 167) or the Signal Find Server (URL: http://signalfind.org/) with TATFIND (URL: http://signalfind.org/tatfind.html; Rose, R.W., T. Brüser,. J. C. Kissinger, and M. Pohlschröder. 2002. Adaptation of protein secretion to extremely high salt concentrations by extensive use of the twin arginine translocation pathway. Mol. Microbiol. 5: 943-950; Dilks, K., W. R. Rose, E. Hartmann, and M. Pohlschorder. 2003. Prokaryotic use of the twin arginine translocation pathway: A Genomic Survey. J. Bacteriol. 185:1478-1483). Moreover, software able to detect conserved protein motifs and families that detects the two Arginine motif may be used, such as the ones present in the PFAM database (PFAM PF10518; URL: http://pfam.xfam.org/family/TAT_signal; Berks BC; A common export pathway for proteins binding complex redox cofactors? Mol Microbiol. 1996;22:393-404).

In some embodiments, the step of identifying sequences having a signal peptide refers to signal peptides for secreted proteins and to transmembrane domains. For example, a bioinformatics tool for predicting a signal peptide (such as SignalP) may be combined with a bioinformatics tool for identifying a transmembrane domain (such as TMHMM) and, as a result, such proteins may be selected, which are predicted have a signal peptide and/or a transmembrane domain (either or both). In other embodiments, the step of identifying sequences having a signal peptide refers to signal peptides for secreted proteins only.

In some embodiments, step (ii) of the method for providing a human microbiota protein drug candidate according to the present invention further comprises an optional sub-step of distinguishing signal peptides of secreted proteins from lipoproteins of gram-negative bacteria.

Such a sub-step of distinguishing signal peptides of secreted proteins from lipoproteins of gram-negative bacteria is preferably performed after the identification of a signal peptide as described above. While the sub-step of distinguishing signal peptides of secreted proteins from lipoproteins of gram-negative bacteria may be performed directly after the identification of a signal peptide, it may also be performed at later stages, e.g. after cleavage of the signal peptide and/or determining the cysteine-content (or rigid proteins).

Signal peptides of lipoproteins of gram-negative bacteria may be similar to signal peptides of secreted proteins. Accordingly, it may be advantageous to distinguish between these two groups (i.e., to identify sequences of lipoproteins of gram-negative bacteria) and to remove sequences of lipoproteins of gram-negative bacteria (or to keep only sequences with signal peptides of secreted proteins, but not with signal peptides of lipoproteins of gram negative bacteria).

Preferred examples of bioinformatics tools (software) to identify bacterial lipoproteins include:
- LipoP (URL: http://www.cbs.dtu.dk/services/LipoP/; Juncker, A. S., Willenbrock, H., von Heijne, G., Brunak, S., Nielsen, H., & Krogh, A. (2003). Prediction of lipoprotein signal peptides in Gram-negative bacteria. Protein Science, 12(8), 1652-1662; O. Rahman, S. P. Cummings, D. J. Harrington and I. C. Sutcliffe: Methods for the bioinformatic identification of bacterial lipoproteins encoded in the genomes of Gram-positive bacteria. World Journal of Microbiology and Biotechnology 24(11):2377-2382 (2008));
- PRED-LIPO (URL: http://biophysics.biol.uoa.gr/PRED-LIPO/; Bagos PG, Tsirigos KD, Liakopoulos TD, Hamodrakas SJ. Prediction of lipoprotein signal peptides in Gram-positive bacteria with a Hidden Markov Model. J Proteome Res. 2008 Dec;7(12):5082-93);
- DOLOP (URL: https://www.mrc-lmb.cam.ac.uk/genomes/dolop/; Babu MM, Priya ML, Selvan AT, Madera M, Gough J, Aravind L, Sankaran K. A database of bacterial lipoproteins (DOLOP) with functional assignments to predicted lipoproteins. J Bacteriol. 2006 Apr;188(8):2761-73);
- LIPPRED (URL: http://www.jenner.ac.uk/LipPred/; Taylor PD, Toseland CP, Attwood TK, Flower DR. LIPPRED: A web server for accurate prediction of lipoprotein signal sequences and cleavage sites. Bioinformation. 2006;1(5):176-179);
- LIPO (URL: http://services.cbu.uib.no/tools/lipo; Berven FS, Karlsen OA, Straume AH, Flikka K, Murrell JC, Fjellbirkeland A, Lillehaug JR, Eidhammer I, Jensen HB. Analysing the outer membrane subproteome of Methylococcus capsulatus (Bath) using proteomics and novel biocomputing tools. Arch Microbiol. 2006 Feb;184(6):362-77); and
- SPEPlip (URL: http://gpcr.biocomp.unibo.it/cgi/predictors/spep/pred_spepcgi.cgi; Piero Fariselli, Giacomo Finocchiaro, Rita Casadio; SPEPlip: the detection of signal peptide and lipoprotein cleavage sites, Bioinformatics, Volume 19, Issue 18, 12 December 2003, Pages 2498-2499).

Preferably, LipoP is used to distinguish between signal peptides of secreted proteins and signal peptides of lipoproteins of gram-negative bacteria. In particular, LipoP performs a prediction over the protein sequence to determine the type of lipoprotein signal peptide encoded. The type I (Spl) is the normal signal peptide, while the type II (Spll) is the lipoprotein signal peptide, which is normally found in proteins located within the periplasm.

It is also preferred that, in addition to a bioinformatics tool for distinguishing between signal peptides of secreted proteins and signal peptides of lipoproteins of gram-negative bacteria the taxonomic assignment of the sequence is then taken into account. If a taxonomic assignment is available for the corresponding gene encoding for that protein, the assignment can be used to discriminate possible lipoproteins. The taxonomical annotation of human gut microbial genes may be performed following the procedure described in Li J, Jia H, Cai X, Zhong H, Feng Q, Sunagawa S, Arumugam M, Kultima JR, Prifti E, Nielsen T, Juncker AS, Manichanh C, Chen B, Zhang W, Levenez F, Wang J, Xu X, Xiao L, Liang S, Zhang D, Zhang Z, Chen W, Zhao H, Al-Aama JY, Edris S, Yang H, Wang J, Hansen T, Nielsen HB, Brunak S, Kristiansen K, Guarner F, Pedersen O, Doré J, Ehrlich SD; MetaHIT Consortium, Bork P, Wang J; MetaHIT Consortium. An integrated catalog of reference genes in the human gut microbiome. Nat Biotechnol. 2014 Aug;32(8):834-41.

### Protein length

Step (ii) of the method for providing a human microbiota protein drug candidate according to the present invention comprises a sub-step, in which the lengths of the sequences are evaluated and sequences having a length in a predetermined length range, namely 20 - 500 amino acids, are selected. Preferably, the proteins have a length of 20 - 350 amino acids, more preferably of 20 - 200 amino acids.

Accordingly, in this step the distribution of protein lengths is evaluated and length cutoffs are established according to the specific group of proteins that are of interest, for example short non-enzymatic proteins and/or longer sequences encoding proteins with functional activities.

In this sub-step, sequences coding for proteins or sequences of proteins are selected, which have a length of 20 or more amino acids. In some instances, sequences coding for proteins or sequences of proteins are selected, which have a length of 25 or more, preferably 30 or more, more preferably 35 or more, even more preferably 40 or more, still more preferably 45 or more, and most preferably 50 or more amino acids are selected. Thereby, small peptides, which may result from annotation biases and artifacts, may be avoided.

Moreover, sequences coding for proteins or sequences of proteins are selected, which have a length of 500 or less amino acids, preferably of 450 or less amino acids, more preferably of 400 or less amino acids, even more preferably of 350 or less amino acids, still more preferably of 300 or less amino acids, and most preferably of 250 or less amino acids, for example no more than 200 or 150 amino acids. Thereby, the maximum length of the selected proteins is limited in order to select relatively small proteins having a size usually found in interacting molecules, such as cytokines, chemokines, growth factors, neuropeptides, peptide hormones, etc..

In some embodiments, sequences coding for proteins or sequences of proteins are selected, which have a length of 25 - 450 amino acids, preferably 30 - 400 amino acids, more preferably 35 - 350 amino acids, even more preferably 40 - 300 amino acids, and still more preferably 45 - 250 amino acids. For example, sequences coding for proteins or sequences of proteins are selected, wherein the proteins have a length of 50 - 200 or 50 - 150 amino acids.

In some embodiments, the length of the protein is assessed in the "mature" protein (in its functional state), i.e. "additional" pre-protein sequences, like signal peptides, which are removed in the mature/functional protein, may be not considered for the length of the protein. To this end, the signal peptide may be identified and removed ("cleaved", e.g. *in silico* with appropriate bioinformatics tools) as described above. Preferably, the length of the human microbiota proteins (without signal peptide) does not exceed 500 amino acids, such as proteins having a length (without signal peptide) of 20 - 500 amino acids. For example, the maximum length of the protein (without signal peptide) may be 400 or 350 amino acids. More preferably, the maximum length of the microbiota protein (without signal peptide) does not exceed 250 amino acids, e.g., not more than 240, 230, 220, 210, 200, 190, 180, 170, 160, 150, 140, 130, 120, 110, 100, 95, 90, 85, 80, 75, 70, 65, 60, 55, 50, 45, 40, 35, 30, 29, 28, 27, 26, 25, 24, 23, 22, 21, or 20 amino acids. The minimum length (without signal peptide) is usually 20 amino acids. In some embodiments, the microbiota protein (without signal peptide) has a length of 20 - 350 amino acids, preferably a length of 20 - 300 amino acids, more preferably a length of 20 - 250 amino acids, even more preferably a length of 20 - 200 amino acids and still more preferably 20 - 150 amino acids, for example a length of 20 to 40, 50, 60, 70, 80, 90, 100, 110, 120, 130 or 140 amino acids.

In other embodiments, the length of the protein is assessed in the "preprotein", i.e. including the signal peptide. Preferably, the length of the human microbiota proteins (with signal peptide) does not exceed 550 amino acids, such as proteins having a length (without signal peptide) of 50 - 550 amino acids. For example, the maximum length of the protein (with signal peptide) may be 500 or 400 amino acids. More preferably, the maximum length of the microbiota protein (with signal peptide) does not exceed 350 amino acids, e.g., not more than 340, 330, 320, 310, 200, 290, 280, 270, 260, 250, 240, 230, 220, 210, 200, 190, 180, 170, 160, 150, 140, 130, 120, 110, 100, 95, 90, 85, 80, 75, 70, 65, 60, 55, or 50 amino acids. The minimum length (including the signal peptide) is usually 50 amino acids. In some embodiments, the microbiota protein (including the signal peptide) has a length of 50 - 400 amino acids, preferably a length of 50 - 350 amino acids, more preferably a length of 50 - 300 amino acids, even more preferably a length of 50 - 250 amino acids and still more preferably 50 - 200 amino acids, for example a length of 50 to 60, 70, 80, 90, 100, 110, 120, 130, 140, 150, 160, 170, 180, or 190 amino acids.

### Cysteine content and structure element conferring a conformational rigid structure

In step (ii) of the method for providing a human microbiota protein drug candidate according to the present invention, such protein sequences or nucleic acid sequences encoding such proteins are identified and selected, wherein the protein or comprises
(a) at least two cysteine residues, and/or
(b) a primary and/or a secondary structure element conferring a conformational rigid structure to the protein.

Such proteins cannot easily change their conformations, i.e. the conformation of the protein is constrained. Thereby, proteins maintain the conformation required for interacting with (e.g., binding to) a target (e.g. a human target of a microbiota protein).

In view thereof, proteins comprising at least two cysteine residues (which may form a cysteine pair), may be identified and selected. Cysteine residues stabilize the protein in the correct conformation. In some embodiments, the protein comprises 3, 4, 5, 6, 7, 8, 9, 10 or more cysteine residues. For example, the microbiota protein may comprise two or three cysteine residues (e.g., forming one cysteine pair), more preferably four or five cysteine residues (e.g., forming two cysteine pairs), even more preferably six or seven cysteine residues (e.g., forming three cysteine pairs), and still more preferably eight or nine cysteine residues (e.g., forming four cysteine pairs). Cysteine residues and in particular disulfide bonds between cysteine pairs confer an increased rigid structure to a protein. Accordingly, it is preferred that sequences of proteins with cysteine motifs, which can result in cysteine bonds, are identified. Therefore, the microbiota protein preferably contains an even number of cysteine residues.

The cysteine content of the microbiota protein may also be calculated in view of the length of the protein. Preferably, the microbiota protein has a cysteine content of at least 1 %. More preferably, the microbiota protein has a cysteine content of at least 2 %. Even more preferably, the microbiota protein has a cysteine content of at least 3 %. Still more preferably, the microbiota protein has a cysteine content of at least 4 %. Most preferably, the microbiota protein (or the fragment or sequence variant thereof) has a cysteine content of at least 5%. For example, the protein (or the fragment or sequence variant thereof) may have a cysteine content of about 6 - 8%, e.g. about 6.5 - 7%.

It is also preferred that the microbiota protein contains an even number of cysteine residues. This increases the chance that all cysteine residues in the protein are involved in the formation of disulfide bonds (also referred to as "disulfide bridge"). However, it is understood that also proteins with uneven numbers of cysteine residues may include disulfide bonds, if they comprise more than two cysteine residues.

In some embodiments, the number of cysteines inside the proteins are determined (counted). For example, the proteins comprising at least two cysteine residues may be identified by counting the number of cysteine residues in the protein.

In other embodiments, the cysteine content of proteins may be identified using a bioinformatics tool, such as KAPPA (http://kappa-sequence-search.sourceforge.net; Joly V, Matton DP. KAPPA, a simple algorithm for discovery and clustering of proteins defined by a key amino acid pattern: a case study of the cysteine-rich proteins. Bioinformatics. 2015 Jun 1;31(11):1716-23). KAPPA can provide the additional information of grouping the proteins according to their cysteines motifs and the similarity of these motifs. Accordingly, KAPPA is particularly useful to identify the presence of specific cysteines motifs within the secreted proteins in addition to the number of cysteines.

In some embodiments, the identification and selection of proteins comprising at least two cysteine residues is performed without taking the amino acids/the sequence of the signal peptide into account, since the signal peptide is usually cleaved, such that a mature protein does not contain a signal peptide. Therefore, the amino acids/the sequence of the signal peptide is preferably disregarded in the identification of proteins comprising at least two cysteine residues. Accordingly, it is preferred that prior to the identification and selection of proteins comprising at least two cysteine residues the sequence of the signal peptide is removed from the protein sequence (*in silico* cleavage). Removal ("cleavage") of the signal peptide may be performed as described above, e.g. by identifying the cleavage site and removing the signal peptide (e.g., *in silico*).

Preferably, the human microbiota protein (in particular without considering the signal peptide) has a length of 20 - 200 amino acids and comprises at least two cysteine residues (e.g., forming one cysteine pair). For example, the microbiota protein (in particular without considering the signal peptide) may have a length of 50 - 150 amino acids and may comprise at least four cysteine residues (e.g., forming two cysteine pairs); or the microbiota protein may have a length of 75 - 150 amino acids and may comprise at least six cysteine residues (e.g., forming three cysteine pairs). More preferably, the microbiota protein (in particular without considering the signal peptide) (i) has a length of 20 - 100 amino acids and comprises at least four cysteine residues (e.g., forming two cysteine pairs); (ii) has a length of 50 - 150 amino acids and comprises at least six cysteine residues (e.g., forming three cysteine pairs); or (iii) has a length of 75 - 200 amino acids and comprises at least eight cysteine residues (e.g., forming four cysteine pairs). Such proteins have a cysteine content of at least 4%. Even more preferably, the microbiota protein (in particular without considering the signal peptide) (i) has a length of 20 - 75 amino acids and comprises at least four cysteine residues (e.g., forming two cysteine pairs); (ii) has a length of 50 - 100 amino acids and comprises at least six cysteine residues (e.g., forming three cysteine pairs); or (iii) has a length of 75 - 125 amino acids and comprises at least eight cysteine residues (e.g., forming four cysteine pairs). Such proteins have a cysteine content of more than 5%.

It is also preferred, that the microbiota protein comprises a primary and/or a secondary structure element conferring a conformational rigid structure. Such primary and/or secondary structure elements may be selected from the group consisting of cysteine motif, leucine-rich repeat (LRR), alpha-helix (a-helix), beta-sheet (β-sheet) and coil. For example, the primary and/or the secondary structure element conferring a conformational rigid structure is a disulfide bridge.

Alternatively, rigid proteins - i.e. proteins comprising a primary and/or a secondary structure element conferring a conformational rigid structure - may be identified *in silico,* in particular by using bioinformatics tools for identification of rigid proteins. Examples of bioinformatics tools for identification of rigid proteins include:
- PSIpred (Predict Secondary Structure) (URL: http://bioinf.cs.ucl.ac.uk/psipred/; Jones DT, Protein secondary structure prediction based on position-specific scoring matrices, J Mol Biol. 1999 Sep 17;292(2):195-202);
- HHblits (Remmert M, Biegert A, Hauser A, Söding J, HHblits: lightning-fast iterative protein sequence searching by HMM-HMM alignment, Nature Methods volume 9, 173-175(2012);
- MADOKA (Deng L, Zhong G, Liu C, Luo J, Liu H, MADOKA: an ultra-fast approach for large-scale protein structure similarity searching, BMC Bioinformatics. 2019 Dec 24;20(Suppl 19):662);
- JPRED (URL: https://www.compbio.dundee.ac.uk/jpred/index.html; Drozdetskiy A, Cole C, Procter J, Barton GJ. JPred4: a protein secondary structure prediction server. Nucleic Acids Res. 2015 Jul 1;43(W1):W389-94);
- SWISS-MODEL (URL: https://swissmodel.expasy.org/; Waterhouse, A., Bertoni, M., Bienert, S., Studer, G., Tauriello, G., Gumienny, R., Heer, F.T., de Beer, T.A.P., Rempfer, C., Bordoli, L., Lepore, R., Schwede, T. SWISS-MODEL: homology modelling of protein structures and complexes. Nucleic Acids Res. 46(W1), W296-W303 (2018); Bertoni, M., Kiefer, F., Biasini, M., Bordoli, L., Schwede, T. Modeling protein quaternary structure of homo- and hetero-oligomers beyond binary interactions by homology. Scientific Reports 7 (2017));
- KINARI (KINematics And Rigidity) and Kinari-2 (URL: http://kinari.cs.umass.edu/Site/index.html; Naomi Fox, Filip Jagodzinski, Yang Li, Ileana Streinu. KINARI-Web: A Server for Protein Rigidity Analysis, Nucleic Acids Research, 39 (Web Server Issue), 2011; Streinu I. Large scale rigidity-based flexibility analysis of biomolecules. Struct Dyn. 2016;3(1):012005); and
- PredyFlexy (URL: https://www.dsimb.inserm.fr/dsimb_tools/predyflexy/; de Brevern AG, Bornot A, Craveur P, Etchebest C, Gelly JC. PredyFlexy: flexibility and local structure prediction from sequence. Nucleic Acids Res. 2012;40(Web Server issue):W317-W322).

For example, the microbiota protein (in particular without considering the signal peptide) may (i) have a length of 20 - 50 amino acids and comprise a primary and/or secondary structure element conferring a conformational rigid structure; or (ii) have a length of 20 - 50 amino acids and comprise a secondary structure element selected from the group consisting of an alpha-helix, a beta-sheet and a coil.

### Additional optional sub-steps

In addition to the selection of sequences according to the above-described three criteria, one or more further sub-steps of step (ii) may be performed. Such optional additional sub-steps include the annotation of sequences and/or the removal of redundant sequences.

In some embodiments, protein or nucleic acid sequences with unknown functions are annotated in step (ii). Accordingly, the method according to the present invention may comprise a sub-step of identifying human microbiota sequences with unknown function and annotating said sequences.

As used herein, the terms "annotation" or "annotating" refer to the attachment of structural and/or biological information to a sequence. In particular, sequence annotation is the process of marking specific features in a nucleic acid sequence or in a protein sequence with descriptive information about structure or function. Accordingly, sequence annotations may describe regions or sites of interest in the protein sequence, such as post-translational modifications, binding sites, enzyme active sites, local secondary structure or other characteristics. For example, genome context information, similarity scores, experimental data, and integrations of other resources may be used to provide sequence annotation.

Annotation facilitates downstream filtering and selection of (predicted) secreted proteins.

Annotation may be performed before, after or in between the other sub-steps of step (ii) as described above. In some embodiments, annotation is performed after
- selecting (a) sequence(s) having, or coding for proteins having, a signal peptide;
- selecting (a) sequence(s) having, or coding for proteins having, a length of 20 - 500 amino acids; and
- selecting (a) sequence(s) comprising at least at least two cysteine residues or (a) sequence(s) comprising, or coding for (a) protein(s) comprising, a primary and/or a secondary structure element conferring a conformational rigid structure.

Annotation may be performed by manual annotation, which involves human expertise, and/or by automatic annotation tools, which perform annotation by computer analysis. For example, an *in silico* method may be used for annotation. In some embodiments, these approaches (manual and automatic) are combined in the same annotation pipeline, for example by complementing each other.

Annotation may be performed by homology-based search tools, such as BLAST, to search for homologous genes or proteins with known structure/function in specific databases. The resulting information is then used to annotate nucleic acid and/or protein sequences.

In general, annotation may provide structural and/or functional information. Structural annotation relates to the identification of structural elements of a nucleic acid or protein sequence, for example ORFs, gene structure, coding regions, and location of regulatory motifs in nucleic acid sequences. More preferred examples of structural annotations relate to structural information on protein level (which may be annotated in both, protein and nucleic acid sequences), such as the identification of protein domains and motifs. Functional annotation relates to the attachment biological information, such as biochemical function, biological function, involved regulation and interactions and information relating to the expression. In the context of the present invention, the annotation preferably comprises or consists of functional annotation. More preferably, the annotation provides a structured view on function.

In general, functional annotation is preferably performed by a homology-based method, a sequence motif-based method, a domain-based method, a structure-based method, a genomic-context based method, by a computational solvent mapping method and/or by a network-based method. More preferably, a combination or two or more of those methods is used.

Preferably, in step (ii) protein or nucleic acid sequences with unknown functions are annotated *in silico.*

Annotation may be performed by a (BLAST-based) comparison against reference database, for example against the Kyoto Encyclopedia of Genes and Genomes (KEGG) and/or against the National Center for Biotechnology Information (NCBI) Reference Sequence Database (RefSeq). RefSeq provides an integrated, non-redundant set of sequences, including genomic DNA, transcripts, and proteins. In KEGG, the molecular-level functions stored in the KO (KEGG Orthology) database may be used. These functions are categorized in groups of orthologs, which contain proteins encoded by genes from different species that evolved from a common ancestor.

Conserved Domain Database (CDD) (https://www.ncbi.nlm.nih.gov/cdd/; Marchler-Bauer A et al. (2011), "CDD: a Conserved Domain Database for the functional annotation of proteins.", Nucleic Acids Res.39(D)225-9) is an alternative database for the annotation of protein sequences with the location of conserved domain footprints, and functional sites inferred from these footprints.

A preferred database for annotation is PFAM (http://pfam.xfam.org/; R.D. Finn, P. Coggill, R.Y. Eberhardt, S.R. Eddy, J. Mistry, A.L. Mitchell, S.C. Potter, M. Punta, M. Qureshi, A. Sangrador-Vegas, G.A. Salazar, J. Tate, A. Bateman: The Pfam protein families database: towards a more sustainable future. Nucleic Acids Research (2016) Database Issue 44: D279-D285; E. L. Sonnhammer, S. R. Eddy, R. Durbin: Pfam: a comprehensive database of protein domain families based on seed alignments. In: Proteins. 28, 1997, S. 405-420).

Further examples of preferred bioinformatics tools (software) for annotation include:
- HMMSCAN (https://www.ebi.ac.uk/Tools/hmmer/search/hmmscan; Finn, R. D., Clements, J. & Eddy, S. R. HMMER web server: interactive sequence similarity searching. Nucleic Acids Res 39, W29-37, doi:10.1093/nar/gkr367 (2011); Eddy, S. R. Accelerated Profile HMM Searches. PLoS Comput Biol 7, e1002195, doi:10.1371/journal.pcbi.1002195 (2011); R.D. Finn, J.Clements, W. Arndt, B.L. Miller, T.J. Wheeler, F. Schreiber, A. Bateman and S.R. Eddy: HMMER web server: 2015 update. Nucleic Acids Research (2015) Web Server Issue 43:W30-W38);
- HAMAP and/or HAMAP-Scan (https://hamap.expasy.org/; https://hamap.expasy.org/hamap_scan.html; Ivo Pedruzzi, Catherine Rivoire, Andrea H. Auchincloss, Elisabeth Coudert, Guillaume Keller, Edouard de Castro, Delphine Baratin, Béatrice A. Cuche, Lydie Bougueleret, Sylvain Poux, Nicole Redaschi, loannis Xenarios, Alan Bridge; HAMAP in 2015: updates to the protein family classification and annotation system, Nucleic Acids Research, Volume 43, Issue D1, 28 January 2015, Pages D1064-D1070; Tania Lima, Andrea H. Auchincloss, Elisabeth Coudert, Guillaume Keller, Karine Michoud, Catherine Rivoire, Virginie Bulliard, Edouard de Castro, Corinne Lachaize, Delphine Baratin, Isabelle Phan, Lydie Bougueleret, Amos Bairoch; HAMAP: a database of completely sequenced microbial proteome sets and manually curated microbial protein families in UniProtKB/Swiss-Prot, Nucleic Acids Research, Volume 37, Issue suppl_1, 1 January 2009, Pages D471-D478);
- iPtgxDB (https://iptgxdb.expasy.org/; U. Omasits, A. R. Varadarajan, et al. An integrative strategy to identify the entire protein coding potential of prokaryotic genomes by proteogenomics. 2017. Genome Research, 27: 2083-2095; A. I. Nesvizhskii. 2014. Proteogenomics: concepts, applications and computational strategies. Nature Methods 11: 1114-1125);
- OrthoDB (http://www.orthodb.org/; Zdobnov EM, Tegenfeldt F, Kuznetsov D, Waterhouse RM, Simão FA, loannidis P, Seppey M, Loetscher A, Kriventseva EV. OrthoDB v9.1: cataloging evolutionary and functional annotations for animal, fungal, plant, archaeal, bacterial and viral orthologs. Nucleic Acids Res. 2017 Jan 4;45(D1):D744-D749);
- UniProt (URL: https://www.uniprot.org/; The UniProt Consortium. UniProt: the universal protein knowledgebase. Nucleic Acids Res. 45: D158-D169 (2017));
- CATH (URL: http://www.cathdb.info/; Ian Sillitoe, Tony E. Lewis, Alison Cuff, Sayoni Das, Paul Ashford, Natalie L. Dawson, Nicholas Furnham, Roman A. Laskowski, David Lee, Jonathan G. Lees, Sonja Lehtinen, Romain A. Studer, Janet Thornton, Christine A. Orengo; CATH: comprehensive structural and functional annotations for genome sequences, Nucleic Acids Research, Volume 43, Issue D1, 28 January 2015, Pages D376-D381);
- PANTHER (URL: http://www.pantherdb.org/; Thomas PD, Campbell MJ, Kejariwal A, Mi H, Karlak B, Daverman R, Diemer K, Muruganujan A, Narechania A. PANTHER: a library of protein families and subfamilies indexed by function. Genome Res. 2003 Sep;13(9):2129-41; Mi H, Muruganujan A, Thomas PD. PANTHER in 2013: modeling the evolution of gene function, and other gene attributes, in the context of phylogenetic trees. Nucleic Acids Res. 2013 Jan;41 (Database issue):D377-86);
- PIRSF (URL: https://pir.georgetown.edu/pirwww/dbinfo/pirsf.shtml; Nikolskaya AN, Arighi CN, Huang H, Barker WC, Wu CH. PIRSF family classification system for protein functional and evolutionary analysis. Evol Bioinform Online. 2007 Feb 10;2:197-209; Wu CH, Nikolskaya A, Huang H, Yeh LS, Natale DA, Vinayaka CR, Hu ZZ, Mazumder R, Kumar S, Kourtesis P, Ledley RS, Suzek BE, Arminski L, Chen Y, Zhang J, Cardenas JL, Chung S, Castro-Alvear J, Dinkov G, Barker WC. PIRSF: family classification system at the Protein Information Resource. Nucleic Acids Res. 2004 Jan 1;32(Database issue):D112-4);
- PRINTS (URL: http://130.88.97.239/PRINTS/index.php; Attwood TK, Coletta A, Muirhead G, Pavlopoulou A, Philippou PB, Popov I, Romá-Mateo C, Theodosiou A, Mitchell AL. The PRINTS database: a fine-grained protein sequence annotation and analysis resource--its status in 2012. Database (Oxford). 2012 Apr 15;2012:bas019; Attwood TK. The PRINTS database: a resource for identification of protein families. Brief Bioinform. 2002 Sep;3(3):252-63; Attwood TK, Beck ME, Bleasby AJ, Parry-Smith DJ. PRINTS--a database of protein motif fingerprints. Nucleic Acids Res. 1994 Sep;22(17):3590-6);
- ProDom (URL: http://prodom.prabi.fr/prodom/current/html/home.php; Kahn, D., Rezvoy, C. and Vivien, F. (2008) Parallel large-scale inference of protein domain families. Proceedings of the 14th International Conference on Parallel and Distributed Systems, December 8-10, 2008, Melbourne, Australia, IEEE, pp. 72-79; Catherine Bru, Emmanuel Courcelle, Sébastien Carrère, Yoann Beausse, Sandrine Dalmar, and Daniel Kahn (2005) The ProDom database of protein domain families: more emphasis on 3D. Nucleic Acids Res. 33: D212-D215; orpet F, Servant F, Gouzy J, Kahn D (2000) ProDom and ProDom-CG: tools for protein domain analysis and whole genome comparisons. Nucleic Acids Res. 28:267-269);
- PROSITE (URL: https://prosite.expasy.org/; Sigrist CJ, de Castro E, Cerutti L, Cuche BA, Hulo N, Bridge A, Bougueleret L, Xenarios I. New and continuing developments at PROSITE. Nucleic Acids Res. 2013 Jan;41(Database issue):D344-7);
- SMART (URL: http://smart.embl-heidelberg.de/; Ivica Letunic, Peer Bork; 20 years of the SMART protein domain annotation resource, Nucleic Acids Research, Volume 46, Issue D1, 4 January 2018, Pages D493-D496; Letunic I, Doerks T, Bork P. SMART: recent updates, new developments and status in 2015. Nucleic Acids Res. 2015 Jan;43(Database issue):D257-60);
- SUPERFAMILY (URL: http://supfam.cs.bris.ac.uk/SUPERFAMILY/; Julian Gough, Kevin Karplus, Richard Hughey, Cyrus Chothia (2001): Assignment of homology to genome sequences using a library of hidden Markov models that represent all proteins of known structure. Edited by G. Von Heijne, Journal of Molecular Biology, Volume 313, Issue 4, Pages 903-919; Derek Wilson, Ralph Pethica, Yiduo Zhou, Charles Talbot, Christine Vogel, Martin Madera, Cyrus Chothia, Julian Gough; SUPERFAMILY-sophisticated comparative genomics, data mining, visualization and phylogeny, Nucleic Acids Research, Volume 37, Issue suppl_1, 1 January 2009, Pages D380-D386); and
- TIGRFAM (URL: http://www.jcvi.org/cgi-bin/tigrfams/index.cgi; Daniel H. Haft, Jeremy D. Selengut, Owen White: The TIGRFAMs database of protein families. Nucleic Acids Res. 2003 Jan 1; 31(1): 371-373; Daniel H. Haft, Brendan, J. Loftus, Delwood L. Richardson, Fan Yang, Jonathan, A. Eisen, Ian T. Paulsen, Owen White: TIGRFAMs: a protein family resource for the functional identification of proteins. Nucleic Acids Res. 2001 Jan 1; 29(1): 41-43).

One or more of these exemplified bioinformatics tools (software) may be used for annotation of sequences.

Preferably, the sequences are annotated by using HMMSCAN and/or PFAM. PFAM is the largest public database of protein family domains and the protein sequences can be searched for these domains using the HMMSCAN software.

In some embodiments, a particular functional domain from a human host molecule is searched within the sequences of the human microbiota proteins, which fulfill all three of the above-mentioned criteria. The human host molecules may be selected from the group consisting of cytokines, chemokines, growth factors, neuropeptides, and peptide hormones.

Alternatively or additionally, the method for providing a human microbiota protein drug candidate according to the present invention may also comprise a sub-step, in which redundant sequences are identified. Optionally, the identified redundant sequences are then removed. In particular, in step (ii) of the method redundant sequences may be identified and removed.

As used herein, the term "redundant sequence" refers to a protein (amino acid) or nucleic acid sequence, which is contained twice (in a dataset). Typically, the double existence of a sequence does not add any further information to a dataset as compared to a single sequence.

Accordingly, such sequences are referred to as "redundant". In particular, the term "redundancy" does not only refer to sequences, which are 100% identical, but also refers to sequences which are largely identical (sequences having at least 95%, preferably at least 90%, more preferably at least 85%, even more preferably at least 80% and most preferably at least 75% sequence identity).

Accordingly, in step (ii) of the method according to the present invention all sequences, which are identical to another sequence are identified and removed. Thereby, the resulting dataset contains each sequence only once. More preferably, in step (ii) of the method according to the present invention sequences having at least 95%, preferably at least 90%, more preferably at least 85%, even more preferably at least 80% and most preferably at least 75% sequence identity to another (selected) sequence are identified and removed.

In the context of the present invention, an amino acid sequence "sharing a sequence identity" of at least, for example, 95% to a query (reference) amino acid sequence, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted, preferably within the above definitions of variants or fragments. The same, of course, also applies similarly to nucleic acid sequences.

For (amino acid or nucleic acid) sequences without exact correspondence, a "% identity" of a first sequence (the subject sequence) may be determined with respect to a second sequence (e.g., the query/reference sequence). In general, the two sequences to be compared may be aligned to give a maximum correlation between the sequences. This may include inserting "gaps" in either one or both sequences, to enhance the degree of alignment. A % identity may then be determined over the whole length of each of the sequences being compared (so-called "global alignment"), that is particularly suitable for sequences of the same or similar length, or over shorter, defined lengths (so-called "local alignment"), that is more suitable for sequences of unequal length.

Methods for comparing the identity (sometimes also referred to as "similarity" or "homology") of two or more sequences are well known in the art. The percentage to which two (or more) sequences are identical can e.g. be determined using a mathematical algorithm. A preferred, but not limiting, example of a mathematical algorithm which can be used is the algorithm of Karlin et al. (1993), PNAS USA, 90:5873-5877. Such an algorithm is integrated in the BLAST family of programs, e.g. BLAST or NBLAST program (see also Altschul et al., 1990, J. Mol. Biol. 215, 403-410 or Altschul et al. (1997), Nucleic Acids Res, 25:3389-3402), accessible through the home page of the NCBI at world wide web site ncbi.nlm.nih.gov) and FASTA (Pearson (1990), Methods Enzymol. 183, 63-98; Pearson and Lipman (1988), Proc. Natl. Acad. Sci. U. S. A 85, 2444-2448.). Sequences which are identical to other sequences to a certain extent can be identified by these programmes. Furthermore, programs available in the Wisconsin Sequence Analysis Package, version 9.1 (Devereux et al., 1984, Nucleic Acids Res., 387-395), for example the programs BESTFIT and GAP, may be used to determine the % identity between two polynucleotides and the % identity and the % homology or identity between two polypeptide sequences. BESTFIT uses the "local homology" algorithm of (Smith and Waterman (1981), J. Mol. Biol. 147, 195-197.) and finds the best single region of similarity between two sequences.

Preferred examples of bioinformatics tools (software) for decreasing redundancy and/or identifying and removing redundant sequences include:
- CD-HIT (URL: http://cd-hit.org; http://weizhongli-lab.org/cd-hit/; Weizhong Li, Lukasz Jaroszewski & Adam Godzik. Clustering of highly homologous sequences to reduce the size of large protein databases. Bioinformatics (2001) 17:282-283; Weizhong Li & Adam Godzik. Cd-hit: a fast program for clustering and comparing large sets of protein or nucleotide sequences. Bioinformatics (2006) 22:1658-1659; Weizhong Li, Limin Fu, Beifang Niu, Sitao Wu and John Wooley. Ultrafast clustering algorithms for metagenomic of CD-HIT-OTU-MiSeqsequence analysis. Briefings in Bioinformatics, (2012) 13 (6): 656-668);
- Decrease Redundancy (URL: https://web.expasy.org/decrease_redundancy/; Gasteiger E, Gattiker A, Hoogland C, Ivanyi I, Appel RD, Bairoch A: ExPASy: The proteomics server for in-depth protein knowledge and analysis. Nucleic Acids Res. 2003 Jul 1; 31(13):3784-8);
- Pisces (URL: http://dunbrack.fccc.edu/PISCES.php; Wang G, Dunbrack RL Jr: PISCES: recent improvements to a PDB sequence culling server. Nucleic Acids Res. 2005 Jul 1; 33(Web Server issue):W94-8; Wang G, Dunbrack RL Jr: PISCES: a protein sequence culling server. Bioinformatics. 2003 Aug 12; 19(12):1589-91);
- BlastClust (BLAST; URL: http://blast.ncbi.nlm.nih.gov; Altschul SF, Gish W, Miller W, Myers EW, Lipman DJ: Basic local alignment search tool. J Mol Biol. 1990 Oct 5; 215(3):403-10; Wheeler D, et al. 2007);
- SkipRedundant (EMBOSS; URL: http://www.bioinformatics.nl/cgi-bin/emboss/skipredundant; Rice P, Longden I, Bleasby A: EMBOSS: the European Molecular Biology Open Software Suite. Trends Genet. 2000 Jun; 16(6):276-7);
- kClust (URL: ftp://toolkit.lmb.uni-muenchen.de/pub/kClust/; Hauser M, Mayer CE, Söding J. kClust: fast and sensitive clustering of large protein sequence databases. BMC Bioinformatics. 2013;14:248);
- UCLUST (URL: drive5.com/usearch/manual/ucluscalgo.html; http://www.drive5.com/usearch/; Edgar, R. C. (2010). "Search and clustering orders of magnitude faster than BLAST". Bioinformatics. 26 (19): 2460-2461); and
- MMSeqs2 (Steinegger, M Söding, MMseqs2 enables sensitive protein sequence searching for the analysis of massive data sets. Nat Biotechnol. 2017 Nov;35(11):1026-1028.).

One or more of these exemplified bioinformatics tools (software) may be used for decreasing redundancy and/or identifying and removing redundant sequences.

Most preferably, CD-HIT is used to remove redundant sequences in the method according to the present invention. CD-HIT is the most widely used sequence clustering software, it is routinely applied in public databases (such as Uniprot). It is particularly preferred that CD-HIT is used with an identity cut-off of 70% or more, for example 75% or 80%.

Removing redundant sequences may be performed before, after or in between the other sub-steps of step (ii) as described above. In some embodiments, removing redundant sequences is performed after
- selecting (a) sequence(s) having, or coding for proteins having, a signal peptide;
- selecting (a) sequence(s) having, or coding for proteins having, a length of 20 - 500 amino acids; and
- selecting (a) sequence(s) comprising at least two cysteine residues or (a) sequence(s) comprising, or coding for (a) protein(s) comprising, a primary and/or a secondary structure element conferring a conformational rigid structure.

Optionally, also the sub-step of annotation may be performed before identifying and removing redundant sequences.

### Preparation of human microbiota proteins (drug candidates)

In step (iii) of the method for providing a human microbiota protein drug candidate according to the present invention the drug candidate, i.e. the human microbiota protein obtained in the selection process of step (ii), is prepared.

In particular, the protein is prepared by recombinant means (i.e., the protein is prepared artificially). For example, the protein may be prepared by chemical synthesis, *in vitro* or expressed by another organism (other than the organism from which the sequence is obtained). Accordingly, the protein obtained by the above method is in particular a recombinant protein. In some embodiments, the human microbiota protein is prepared in step (iii) by chemical synthesis. In some embodiments, the human microbiota protein is prepared in step (iii) by *in vitro* synthesis (cell-free expression) or by recombinant overexpression. Preferably, the protein is purified.

The skilled person is aware of various methods to prepare a protein based on the amino acid sequence or the nucleic acid sequence. For example, the protein may be prepared by chemical synthesis, *in vitro* synthesis (cell-free expression) or by (*in vivo*) recombinant (over)expression.

*In vitro* protein synthesis (also referred to as *"in vitro* protein expression", "cell-free protein expression" and "cell-free protein synthesis") is the production of recombinant proteins in solution using biomolecular translation machinery extracted from cells. *In vitro* protein synthesis occurs in cell lysates or in cocktails of recombinant proteins rather than within cultured cells and, thus, it is achieved without the use of living cells. The *in vitro* protein synthesis environment is not constrained by a cell wall or homeostasis conditions necessary to maintain cell viability. Accordingly, *in vitro* protein synthesis enables direct access and control of the translation environment. Moreover, this technique enables rapid expression and manufacture of functional proteins. *In vitro* protein synthesis is useful for various applications including optimization of protein production, optimization of protein complexes, to study protein synthesis, incorporating non-natural amino acids, high-throughput screens, functional analyses, molecular interaction detection, molecular structure and localization analyses and molecular diagnostics.

Common components of a cell-free reaction comprise proteins necessary to achieve *in vitro* transcription and transduction, such as cocktails of recombinant proteins or cell extracts. In addition, an energy source, a supply of amino acids, a nucleic acid encoding the protein to be expressed and, optionally, a cofactor, such as magnesium, may be added. *In vitro* protein synthesis can be accomplished with several kinds and species of cell extracts or with cocktails of recombinant proteins. A cell extract may be obtained, for example, by lysing a cell of interest and centrifuging out the cell walls, DNA genome, and other debris, such that the necessary cell machinery (including ribosomes, aminoacyl-tRNA synthetases, translation initiation and elongation factors, nucleases, etc.) remains. Examples include cell extracts made from *E. coli* (ECE)*,* rabbit reticulocytes (RRL), wheat germ (WGE), insect cells (ICE, for example SF9 or SF21) and human cells, which are all commercially available. Examples of commercially available *in vitro* protein synthesis systems include, but are not limited to, RTS (5 PRIME); Expressway™ (Life Technologies); S30 T7 high yield (Promega); One-step human IVT (Thermo Scientific); WEPRO® (CellFree Sciences); TNT® coupled (Promega); RTS CECF (5 PRIME); TNT® Coupled (Promega); Retic lysate IVT™ (Life Technologies); TNT® T7 (Promega); EasyXpress Insect kit(Qiagen/RiN A); PURExpress® (New England Biolabs); and PURESYSTEM® (BioComber)). In some embodiments, PURExpress® (New England Biolabs) may be used, which is a cocktail of recombinant proteins necessary to achieve in vitro transcription and transduction. In other embodiments, bacterial extracts, in particular ECE (*E*. *coli* extract), may be used, since they provide the bacterial machinery (which is ideal for expression of bacterial proteins) and typically achieve high yields. A more detailed description of *in vitro* protein synthesis in bacterial extracts may be derived from Hani S. Zaher, Rachel Green: Chapter One - In Vitro Synthesis of Proteins in Bacterial Extracts, Editor(s): Jon Lorsch, Methods in Enzymology, Academic Press, Vol. 539, 2014, p. 3-15, ISSN 0076-6879, ISBN 9780124201200.

The nucleic acid used in *in vitro* protein synthesis is preferably RNA or DNA. For example, isolated RNA (in particular mRNA) synthesized *in vivo* or *in vitro* may be used as template for translation. It is also preferred to use DNA, in particular a coupled translation/transcription system, in which circular or linear DNA, such as a gene/an ORF cloned into a plasmid vector (cDNA), or a linear DNA template, such as a PCR-generated template, are used. The nucleic acid may be codon optimized. In some embodiments, direct synthesis of DNA is used, preferably with codon optimization.

The nucleic acid, e.g., a synthetic DNA molecule, may be subcloned into a vector. The vector may be an expression vector, which may be used for production of expression products such as peptides, polypeptides or proteins. For example, an expression vector may comprise sequences needed for transcription of a sequence stretch of the vector, such as a promoter sequence (e.g., a T7 promoter). Accordingly, the vector may comprise a (T7) promoter. In addition, the vector may contain a particular tag, if required.

*In vitro*/cell-free protein synthesis is preferably applied to proteins having a minimum length of 20 amino acids, preferably having a minimum length of 30 amino acids, more preferably having a minimum length of 40 amino acids, even more preferably having a minimum length of 45 amino acids and still more preferably having a minimum length of 50 amino acids. For example, proteins having a length (preferably without signal peptide) of 50 to 350 amino acids or 50 to 500 amino acids are synthesized by *in vitro*/cell-free protein synthesis as described above.

Alternatively, the protein may be prepared by chemical synthesis, as known in the art and described, for example, by Fields GB. Introduction to peptide synthesis. Curr Protoc Protein Sci. 2002;Chapter 18:Unit-18.1. doi:10.1002/0471140864.ps1801s26. For example, solid phase technologies known in the art (solid-phase peptide synthesis (SPPS)) may be used, e.g. applying Fmoc-based chemistries. SPPS allows the rapid assembly of a peptide chain through successive reactions of amino acid derivatives on an insoluble porous support. Various commercial suppliers of chemical protein and peptide synthesis are available, e.g. Pepscan (Lelystad, Netherlands), GenScript (Piscataway, NJ, USA), LifeTein (Somerset, NJ, USA), JPT (Berlin, Germany).

Chemical synthesis, e.g. SPPS, may be applied to proteins having a maximum length of 100 amino acids, preferably having a maximum length of 90 amino acids, more preferably having a maximum length of 80 amino acids, even more preferably having a maximum length of 70 or 60 amino acids and still more preferably having a maximum length of 50 amino acids. For example, proteins having a length (preferably without signal peptide) of 15 to 50 amino acids or 20 to 50 amino acids are synthesized by chemical synthesis, e.g. SPPS.

Accordingly, longer proteins, e.g. having a length as described above (e.g., 50 - 350 amino acids), may be synthesized by *in vitro*/cell-free protein synthesis and shorter proteins, e.g. having a length as described above (e.g., 20 - 50 amino acids) may be synthesized by chemical synthesis, e.g. SPPS.

The protein may also be prepared by *in vivo* recombinant (over)expression. This is typically achieved by the manipulation of gene expression in an organism such that it expresses large amounts of a recombinant nucleic acid, in particular a recombinant gene. Accordingly, this method involves living cells and is a cell-based system. This process comprises in particular the transcription of the recombinant DNA to messenger RNA (mRNA) and the translation of mRNA into polypeptide chains, which may be folded into functional proteins. The proteins may then be targeted to specific subcellular or extracellular locations.

As used herein, the term "overexpression" refers to a (excessively) high level of gene expression, which may produce a pronounced gene-related phenotype. In a recombinant cell-based expression system, a foreign nucleic acid is introduced into a cell for expression. Many ways to introduce a foreign nucleic acid into a cell are known in the art. Examples of nucleic acid sources and delivery mechanisms include viruses (such as baculovirus, retrovirus, adenovirus), plasmids, artificial chromosomes and bacteriophage (such as lambda). Moreover, the skilled person is aware of many different host systems. Examples include bacteria (such as *E.coli, B. subtilis*), yeast (such as *S.cerevisiae*), filamentous fungi (such as *Aspergillus, Trichoderma,* and *Myceliophthora thermophila* C1), insect cells (such as SF9, SF21 and High Five-strains), and mammalian cell lines (HeLa, CHO, HEK293, Crucell's Per.C6, Glycotope and CEVEC).

Bacterial host cells are most preferred, since they provide the bacterial machinery (which is ideal for expression of bacterial proteins) and typically achieve high yields. Preferred examples of bacterial hosts include *E.coli, B. subtilis, Corynebacterium and Pseudomonas fluorescens. P. fluorescens* and *E. coli are* most preferred. *P. fluorescens* is a metabolically versatile organism, allowing for high throughput screening and rapid development of complex proteins. *P. fluorescens* is most well-known for its ability to rapid and successfully produce high titers of active, soluble protein. The techniques for overexpression in *E*. *coli* are well-known in the art and work by increasing the number of copies of the gene or increasing the binding strength of the promoter region so assisting transcription. DNA is preferably introduced using a plasmid expression vector.

Accordingly, in some embodiments of the method for preparing a protein of the human microbiota metasecretome according to the present invention a nucleic acid molecule is used as template for preparing the protein. In this context, the method for preparing a protein of the human microbiota metasecretome according to the present invention (in particular wherein a nucleic acid molecule is used as template for preparing the protein) may comprise a step of
- Identifying a start and a stop codon in the nucleic acid sequence.

The start codon is the first codon of a messenger RNA (mRNA) transcript translated by a ribosome. The start codon always codes for methionine in eukaryotes and a modified Met (fMet) in prokaryotes. The most common start codon is AUG (or ATG in the corresponding DNA sequence). The start codon is often preceded by a 5' untranslated region (5' UTR). In prokaryotes the 5'UTR typically includes the ribosome binding site. Alternative start codons are different from the standard AUG codon and are found in both prokaryotes (bacteria) and eukaryotes. Alternate start codons are still translated as Met when they are at the start of a protein (even if the codon encodes a different amino acid otherwise). For example, E. coli uses 83% AUG (ATG), 14% GUG (GTG), 3% UUG (TTG) and one or two others (e.g., an AUU (ATT) and possibly a CUG (CTG)).

The stop codon (or termination codon) is a nucleotide triplet within messenger RNA that signals a termination of translation into proteins. Stop codons signal the termination of this process by binding release factors, which cause the ribosomal subunits to disassociate, releasing the amino acid chain. In particular, the stop codon consists of the nucleic acid sequence UAA (TAA), UAG (TAG), or UGA (TGA).

Preferably, identification of a start codon is performed *in silico.* Bioinformatics programs usually allow for alternate start and stop codons when searching for protein coding genes.

It is also preferred that the human microbiota protein prepared in step (iii) is synthetized without the signal peptide identified in step (ii). Accordingly, in particular if not already performed in step (ii) as described above, step (iii) may involve a step of removing the signal peptide from the protein or nucleic acid sequence (e.g., as described above). This is to ensure that in further analyses only the mature protein is used. Moreover, for certain analyses, amino acid frequency calculations are required, which are typically performed without signal peptide to arrive at the correct values.

Removal of the signal peptide may be done, for example, *in vivo* or *in silico.* in some embodiments, the signal peptide is removed *in vivo,* for example in cell-based recombinant expression systems due to the cell's machinery after delivery/translocation.

It is also preferred that the signal peptide is removed *in silico,* in particular before preparation of the protein. Thereby, correct synthesis and folding of the protein, in particular for *in vitro* protein synthesis, are ensured. In general, *in silico* removal/cleavage of the signal peptide may be performed as described above, in particular by using the bioinformatics tools as described above.

In some embodiments, the method for preparing a protein of the human microbiota metasecretome according to the present invention may further comprise a step of
- checking the prepared protein of the human microbiota metasecretome.

For example, it may be checked whether the prepared protein is correctly folded and/or whether the prepared protein has the correct size and/or conformation.

A preferred method for checking the prepared protein is a Western Blot. A Western Blot provides information regarding the size of the protein and, for example, whether the antibody used in the Western Blot can bind to the protein (thereby showing that at least the epitope has the correct conformation). Other examples for methods to check whether the protein is properly folded include nuclear magnetic resonance spectroscopy (NMR), X-ray crystallography, fluorescence spectroscopy, circular dichroism, vibrational circular dichroism of proteins, dual polarization interferometry and atomic force microscopy (AFM). Proteolysis assays may be used to quantify stability of proteins.

In some embodiments, the obtained proteins may be quantified. For example, the obtained proteins may contain an appropriate tag, such as 6HIS or Hibit and the level of proteins may be determined by using a quantitative method, such as alphalisa or HTRF with competitive quantitative assay, e.g. where a HIStag-biotyn probe is competed. If Hibit is used, the level of protein may be determined by using a Hibit luciferase assay.

### Determining biological activity, in particular relating to interaction with the host

Preferably, the method for providing a human microbiota protein drug candidate according to the invention includes an additional step (iv), wherein at least one biological activity of the human microbiota protein drug candidate is determined, in particular which relates to an interaction with the human host and/or to any (other) interactions of interest such as bacterial-bacterial interactions. Such a step (iv) may be performed directly or indirectly following step (iii) of preparing said protein.

As used herein, the term "interaction" refers to the physical contacts of high specificity established between two or more (protein) molecules as a result of biochemical events steered by electrostatic forces including the hydrophobic effect.

In general, interactions may be tested by a variety of methods known in the art, for example *in silico, in vitro, in vivo etc.*

In some embodiments, the biological activity, e.g. the interaction, may be tested *in silico.* Many suitable *in silico* methods for interaction testing are known in the art. For example, many structural protein motifs are known in the art and can be predicted by appropriate software/bioinformatics tools, for example those described above for annotation.

Preferred examples of *in silico* methods include:
- phylogenetic profiling (which finds pairs of protein families with similar patterns of presence or absence across large numbers of species; this method may be applied on protein level or on the level of protein domains);
- prediction of co-evolved protein pairs based on similar phylogenetic trees (which uses the phylogenetic trees of protein pairs to determine if interactions exist by searching for homologues of the protein of interest);
- the Rosetta stone method (used, for example, by STRING; Date SV. The Rosetta stone method. Methods Mol Biol. 2008;453:169-80);
- classification methods (which use data to train a program (classifier) to distinguish positive examples of interacting protein/domain pairs with negative examples of non-interacting pairs);
- interference of interactions from homologues structures (which uses known protein complex structures to predict and structurally model interactions between query protein sequences);
- association methods (which look for characteristic sequences or motifs that can help distinguish between interacting and non-interacting pairs, namely for sequence-signatures that are found together more often than by chance);
- identification of structural patterns (which builds a library of known protein-protein interfaces from the Protein Data Bank (PDB), where the interfaces are defined as pairs of polypeptide fragments that are below a threshold slightly larger than the Van der Waals radius of the atoms involved);
- Bayesian network modeling (which integrate data from a wide variety of sources, including both experimental results and prior computational predictions, and use these features to assess the likelihood that a particular potential protein interaction is a true positive result), and
- Domain-pair exclusion analysis

Preferred examples of bioinformatics tools/software to predict/test the interaction of the identified/prepared protein of the human microbiota metasecretome with a human host protein include:
- STRING (URL: https://string-db.org/; Szklarczyk D, Morris JH, Cook H, Kuhn M, Wyder S, Simonovic M, Santos A, Doncheva NT, Roth A, Bork P, Jensen LJ, von Mering C. The STRING database in 2017: quality-controlled protein-protein association networks, made broadly accessible. Nucleic Acids Res. 2017 Jan 45:D362-68; von Mering C, Huynen M, Jaeggi D, Schmidt S, Bork P, Snel B. STRING: a database of predicted functional associations between proteins. Nucleic Acids Res. 2003 Jan; 31:258-61);
- Prediction of Interaction Specificity in Two-component Systems (URL: http://www.swissregulon.unibas.ch/cgi-bin/TCS.pl; Lukas Burger and Erik van Nimwegen (2008): Accurate prediction of protein-protein interactions from sequence alignments using a Bayesian method. Molecular Systems Biology 4:165);
- Mentha (URL: http://mentha.uniroma2.it/; Alberto Calderone, Luisa Castagnoli & Gianni Cesareni (2013): mentha: a resource for browsing integrated protein-interaction networks. Nature Methods 10, 690);
- GPS-Prot (URL: http://gpsprot.org/index.php; Fahey ME, Bennett MJ, Mahon C, Jäger S, Pache L, Kumar D, Shapiro A, Rao K, Chanda SK, Craik CS, Frankel AD, Krogan NJ. GPS-Prot: a web-based visualization platform for integrating host-pathogen interaction data. BMC Bioinformatics. 2011 Jul 22;12:298);
- COMPASS (URL: http://prodata.swmed.edu/compass/compass.php; R.I. Sadreyev, M. Tang, B. Kim and N.V. Grishin (2009) COMPASS server for homology detection: improved statistical accuracy, speed, and functionality. Nucleic Acids Res doi:10.1093/nar/gkp360);
- PSICQUIC (URL: http://www.ebi.ac.uk/Tools/webservices/psicquic/view/home.xhtml; Aranda B, Blankenburg H, Kerrien S, Brinkman FS, Ceol A, Chautard E, Dana JM, De Las Rivas J, Dumousseau M, Galeota E, Gaulton A, Goll J, Hancock RE, Isserlin R, Jimenez RC, Kerssemakers J, Khadake J, Lynn DJ, Michaut M, O'Kelly G, Ono K, Orchard S, Prieto C, Razick S, Rigina O, Salwinski L, Simonovic M, Velankar S, Winter A, Wu G, Bader GD, Cesareni G, Donaldson IM, Eisenberg D, Kleywegt GJ, Overington J, Ricard-Blum S, Tyers M, Albrecht M, Hermjakob H. PSICQUIC and PSISCORE: accessing and scoring molecular interactions. Nat Methods. 2011 Jun 29;8(7):528-9);
- Struct2Net (URL: http://cb.csail.mit.edu/cb/struct2net/webserver/; R. Hosur, J. Peng, A. Vinayagam, U. Stelzl, J. Xu, N. Perrimon, J. Beinkowska, and B. Berger. 2012. "Coev2Net: a computational framework for boosting confidence in high-throughput protein-protein interaction datasets." Genome Biology, doi:10.1186/gb-2012-13-8-r76; R. Singh, D. Park, J. Xu, R. Hosur, and B. Berger. 2010. "Struct2Net: a Web-Service to Predict Protein-Protein Interactions Using a Structure-based Approach." Nucleic Acids Research, doi:10.1093/nar/gkq481);
- APID (Agile Protein Interactomes Dataserver; URL: http://cicblade.dep.usal.es:8080/APID/init.action; Alonso-Lopez D, Gutiérrez MA, Lopes KP, Prieto C, Santamaria R, De Las Rivas J. APID interactomes: providing proteome-based interactomes with controlled quality for multiple species and derived networks. Nucleic Acids Research 2016; doi: 10.1093/nar/gkw363; Prieto C, De Las Rivas J. APID: Agile Protein Interaction DataAnalyzer. Nucleic Acids Res. 2006, 34(Web Server issue):W298-302);
- lnteractome3D (URL: https://interactome3d.irbbarcelona.org/; Mosca R, Céol A, Aloy P, Interactome3D: adding structural details to protein networks, Nature Methods (2013) 10(1):47-53, doi:10.1038/nmeth.2289); and
- InterPreTS (URL: http://www.russelllab.org/cgi-bin/tools/interprets.pl; Aloy P, Russell RB. InterPreTS: protein interaction prediction through tertiary structure. Bioinformatics. 2003 Jan;19(1):161-2).

In some embodiments, the structure of the human microbiota protein may be determined (e.g., *in silico*) and compared to the (known) structure of one or more human host molecules, in particular one or more human host proteins. Thereby, for example microbiome mimics of human host molecules can be identified. The human host molecules may be selected from the group consisting of cytokines, chemokines, growth factors, neuropeptides, peptide hormones. In some embodiments, a particular functional domain from a human host molecule may be searched within the sequences of the human microbiota proteins, which fulfill all three of the above-mentioned criteria.

Preferably, the interaction of the identified/prepared protein of the human microbiota metasecretome with a human host molecule, in particular a human host protein is tested *in vitro* or *in vivo.* Accordingly, a biological activity (e.g., based on an interaction with a human system, human cell or human molecule) of the identified/prepared protein of the human microbiota metasecretome may be determined *in vitro* or *in vivo.* In this context, screening methods may be used to investigate the involvement of the obtained proteins (e.g., a protein library) in biological mechanisms (metabolism, immunity, cellular integrity). For example, the obtained proteins (e.g., a protein library) may be screened on tests dedicated to G-protein coupled receptors (GPCRs), e.g. by generic assays, such as cAMP release into the cells or IP3 generation (by using competitive alphascreen or HTRF method). Another example of a useful readout of biological activity of an obtained protein may be the induction of calcium release from human cells.

In some embodiments, the effects of the obtained proteins on immunity may be tested, e.g. by using human immune cells, such as peripheral blood mononuclear cells (PBMCs) or a selected subset of human immune cells (e.g., macrophages, dendritic cells (DCs), T cells (e.g., subtype-specific)). In this context, for example cytokine release from human cells exposed to the obtained protein may be determined, e.g. by methods as Elisa, Alphascreen, or HTRF.

In some embodiments, the biological activity may be tested by checking for (human) epithelial cells injury by testing viability and trans-epithelial/endothelial resistance (TEER); by checking for the expression of specific target genes by using qPCR or screening on promoter assays; or by checking for potential ligand / modulators of check point molecules, e.g., by using PPI assays with commercial assays based on alphalisa / alphascreen or HTRF technology.

Additionally (to testing the biological activity; e.g., after testing the biological activity) or alternatively, the interaction with the human system may be tested by investigating protein-protein interactions (PPI), in particular of (i) the identified/obtained protein of the human microbiota metasecretome and (ii) a human protein. For example, the method for testing protein-protein interactions may be selected from the group consisting of affinity purification/mass spectrometry, co-immunoprecipitation, bimolecular fluorescence complementation, affinity electrophoresis, label transfer, phage display, tandem affinity purification, photo-reactive amino acid analogues (*in vivo*), SPINE, quantitative immunoprecipitation combined with knock-down (QUICK), bio-layer interferometry, Dual polarisation interferometry (DPI), Static light scattering (SLS), Dynamic light scattering (DLS), Surface plasmon resonance, Fluorescence polarization/anisotropy, fluorescence correlation spectroscopy, fluorescence cross-correlation spectroscopy (FCCS), Fluorescence resonance energy transfer (FRET), NMR, Isothermal titration calorimetry (ITC), Microscale thermophoresis (MST), Rotating cell-based ligand binding assay, Single color reflectometry (SCORE), protein-fragment complementation assays (PCA), microarrays in particular peptide arrays and protein arrays, far-western blot analysis, and display methods such as peptide phage display, yeast surface display, yeast two-hybrid and bacterial two-hybrid screen, and any combination thereof. Peptide microarrays, protein arrays, peptide phage display, yeast surface display, yeast two-hybrid and bacterial two-hybrid screen are particularly suitable for high-throughput approaches.

For example, an *in silico* technique as described above may be used for predicting the interaction of the identified/prepared protein of the human microbiota metasecretome with a human host molecule, in particular a human host protein, and the interaction may then be tested experimentally (for example as described above), e.g. by biochemical or biological experiments, e.g. *in vitro* or *in vivo.*

Preferably, in the method for providing a human microbiota protein drug candidate according to the present invention the identified protein is a secretagogue. Secretagogues are substances that cause other substances to be secreted. The substances to be secreted may be selected from the group consisting of cytokines (e.g. interleukins), chemokines, growth factors, neuropeptides, and peptide hormones. For example, the identified human microbiota protein drug candidate may be a secretagogue inducing the secretion of Interleukin-10 (IL-10) from human immune cells.

Moreover, the identified human microbiota protein drug candidate may be immunomodulatory.

As used herein, the terms "immunomodulatory" and "immunomodulation" refers to the modification (e.g., induction, amplification, attenuation, prevention, or reduction) of an immune response. Thereby, it is in particular referred to an immune response of the host, i.e. a human immune response.

### Library and database of human microbiota protein drug candidates

In a further aspect the present invention also provides a library of human microbiota protein drug candidates obtainable by the method for providing a human microbiota protein drug candidate as described above.

In general, the library may be a protein library, i.e. a library containing human microbiota protein drug candidates, or a nucleic acid library, in particular a DNA library, which contains nucleic acid molecules (e.g., cloned DNA fragments) encoding the human microbiota protein drug candidates. In some embodiments, the library is a protein library comprising the human microbiota protein drug candidates. In other embodiments, the library is a DNA library comprising DNA molecules encoding the human microbiota protein drug candidates. In order to obtain a DNA library, DNA molecules encoding the human microbiota protein drug candidates may be synthesized (or extracted) and cloned into appropriate vectors (e.g., plasmids or the genome of a bacteriophage).

Accordingly, the present invention also provides a method for preparing a library of human microbiota protein drug candidates. The human microbiota protein drug candidates are identified as described above and, thereafter, a DNA or protein library is prepared, e.g. by collection of the prepared proteins or by preparing a DNA library as described above and known in the art.

Moreover, the present invention also provides a database of human microbiota protein drug candidates obtainable by the method for providing a human microbiota protein drug candidate as described above.

In general, the database contains the protein and/or nucleic acid sequences of the human microbiota protein drug candidates obtainable by the method for providing a human microbiota protein drug candidate as described above. In addition, the database may also contain further information, for example regarding annotation and/or biological activity. A database may easily be obtained based on the sequences selected as human microbiota protein drug candidates in step (ii) of the method for providing a human microbiota protein drug candidate as described above.

Accordingly, the present invention also provides a method for preparing a database of human microbiota protein drug candidates. The human microbiota protein drug candidates are identified/selected as described above and the database provides a respective collection of protein and/or nucleic acid sequences of human microbiota protein drug candidates.

### Method for identification of proteins of the human microbiome metasecretome

In a further aspect the present invention also provides a method for identification of a protein of the human microbiota metasecretome, the method comprising the following steps:
(i) providing a plurality of human microbiota protein sequences and/or a plurality of nucleic acid sequences encoding a plurality of human microbiota proteins;
(ii) identifying in the sequences provided in step (i) one or more sequence(s) of (a) protein(s) of the human microbiota metasecretome, wherein the sequence(s) of the protein of the human microbiota metasecretome is/are selected according to the following criteria:
   - (a) sequence(s) having, or coding for proteins having, a signal peptide;
   - (a) sequence(s) having, or coding for proteins having, a length of 20 - 500 amino acids; and
   - (a) sequence(s) comprising at least two cysteine residues or (a) sequence(s) comprising, or coding for (a) protein(s) comprising, a primary and/or a secondary structure element conferring a conformational rigid structure.

Steps (i) and (ii) of the method for identification of a protein of the human microbiota metasecretome correspond exactly to steps (i) and (ii) of the method for providing a human microbiota protein drug candidate of the invention as described above. The "protein of the human microbiota metasecretome" corresponds to the "human microbiota protein drug candidate" as described in the method above. Accordingly, the detailed description, embodiments and examples as outlined above for the method for providing a human microbiota protein drug candidate of the invention apply accordingly to the method for identification of a protein of the human microbiota metasecretome. The only difference between those methods is that for the method for identification of a protein of the human microbiota metasecretome step (iii) of preparing the protein is not mandatory (while it is for the above-described method for providing a human microbiota protein drug candidate).

In an exemplified method for identification of a protein of the human microbiota metasecretome according to the present invention, step (ii) may comprise the following substeps (preferably in the order as mentioned):
1. a plurality of sequences having a signal peptide is identified using Phobius,
2. annotation is performed using HMMSCAN and PFAM,
3. identification and removal of redundant sequences is performed using CD-HIT, preferably at an identity cut-off of 75%,
4. sequences coding for proteins having a length of 50 - 350 amino acids or sequences of proteins having a length of 50 - 350 amino acids are selected;
5. sequences having a signal peptide are identified using SignalP; and
6. sequences comprising at least two cysteine residues or sequences comprising, or coding for proteins comprising, a primary and/or a secondary structure element conferring a conformational rigid structure are selected.

As outlined above, the detailed description of the method for providing a human microbiota protein drug candidate of the invention applies accordingly.

In a further aspect, the present invention also provides a method for generating a human microbiota metasecretome sequence database, the method comprising the identification of a plurality of human microbiota metasecretome (protein and/or nucleic acid) sequences as described above, i.e. according to the method for identification of a protein of the human microbiota metasecretome according to the present invention.

In particular, the method for generating a human microbiota metasecretome sequence database comprises a step of compiling a plurality of human microbiota metasecretome protein sequences.

The database may comprise nucleic acid sequences encoding metasecretome proteins and/or protein (amino acid) sequences. Preferably, the database comprises protein (amino acid) sequences. More preferably, the database comprises protein (amino acid) sequences and the (corresponding) nucleic acid sequences.

In addition, the database may comprise annotation information, preferably obtained (i) in combination with the original sequences (e.g., from other databases, such as ENSEMBL, UniProt etc.) and/or (ii) by performing an annotation step in the method for identification of a protein of the human microbiota metasecretome according to the present invention as described above.

In the method for generating a human microbiota metasecretome sequence database according to the present invention it is preferred that in step (i) of the method for identification of a protein of the human microbiota metasecretome according to the present invention a plurality of human microbiota protein sequences and/or a plurality of nucleic acid sequences encoding a plurality of human microbiota proteins is provided and compiled in a database and wherein the subsequent steps (i.e., the sub-steps of step (ii)) are performed on the database or on the sequences contained in the database.

In other words, it is preferred that a first database is compiled in step (i) of the method for identification of a protein of the human microbiota metasecretome according to the present invention and that each and every of the subsequent steps are performed on the database or on the sequences contained in the database.

Preferably, a relational database management system (RDBMS) is used. The term "relational database management system (RDBMS)" refers to a database management system (DBMS) that is based on the relational model (Codd E.F. (1970): A Relational Model of Data for Large Shared Data Banks; Communications of the ACM; 13(6): 377-387).

Preferred examples of relational database management systems include:
- MySQL (URL: https://www.mysql.com/; Oracle Corporation, Redwood City, USA)
- Oracle Database (URL: https://www.oracle.com/database/index.html; Oracle Corporation, Redwood City, USA);
- Microsoft SQL server (URL: https://www.microsoft.com/en-us/sql-server; Microsoft corporation);
- PostgreSQL (URL: https://www.postgresql.org/; The PostgreSQL Global Development Group); and
- IBM DB2 (URL: https://www.ibm.com/analytics/us/en/db2/; IBM).

### Method for preparing a protein of the human microbiome metasecretome

In a further aspect, the present invention also provides a method for preparing a protein of the human microbiota metasecretome comprising the identification of a plurality of human microbiota metasecretome protein sequences and/or of a plurality of nucleic acid sequences encoding a plurality of human microbiota metasecretome proteins according to the present invention as described above followed by a step of
- preparing one or more protein(s) of the human microbiota metasecretome based on the previously identified sequence(s).

This method corresponds to the method for providing a human microbiota protein drug candidate of the invention as described above. Namely, the identification of a plurality of human microbiota metasecretome protein sequences and/or of a plurality of nucleic acid sequences encoding a plurality of human microbiota metasecretome proteins corresponds to steps (i) and (ii) of the method for providing a human microbiota protein drug candidate of the invention as described above. The step of preparing one or more protein(s) of the human microbiota metasecretome based on the previously identified sequence(s) corresponds to step (iii) of the method for providing a human microbiota protein drug candidate of the invention as described above. The "protein of the human microbiota metasecretome" corresponds to the "human microbiota protein drug candidate" as described in the method above. Accordingly, the detailed description, embodiments and examples as outlined above for the method for providing a human microbiota protein drug candidate of the invention apply accordingly to the method for preparing a protein of the human microbiota metasecretome.

In a further aspect the present invention also provides a method for identifying and/or providing a protein of the human microbiota metasecretome interacting with a human host molecule comprising
- the method for identification of a plurality of human microbiota metasecretome protein sequences and/or of a plurality of nucleic acid sequences encoding a plurality of human microbiota metasecretome proteins according to the present invention as described above,
- and, optionally, the method for preparation of one or more protein(s) of the human microbiota metasecretome according to the present invention as described above,
followed by a step of testing the interaction of the identified/prepared protein of the human microbiota metasecretome with a human host molecule, in particular a human host protein.

The additional step in this method corresponds to step (iv) in the method for providing a human microbiota protein drug candidate of the invention as described above. Accordingly, the detailed description, embodiments and examples as outlined above for the method for providing a human microbiota protein drug candidate of the invention apply accordingly to the method for identifying and/or providing a protein of the human microbiota metasecretome interacting with a human host molecule.

In a further aspect, the present invention also provides a human microbiota metasecretome protein obtainable by the methods according to the present invention as described above for use in medicine. Such microbiota proteins are usually secreted or located on the cell surface, they have a length of 20 - 500 amino acids and have at least two cysteine residues and/or a rigid structure, as described above. The specific details, e.g. preferred embodiments, described above in the context of the methods of the invention with regard to the human microbiota metasecretome protein apply to this aspect accordingly.

Namely, the proteins of the human microbiota metasecretome, which may be obtained according to the methods of the present invention as described above are useful in preventive and therapeutic applications in humans. This is supported by various reports showing that modulation of microbiome protein expression levels and expression in specific conditions or diseases suggest a biological effect on the human host.

In a preferred embodiment, the human microbiota metasecretome protein was predicted and/or determined to interact with a human host molecule, in particular a human host protein as described above. Such interactions suggest that microbiome proteins mimic the functions of human proteins and may thus be useful in medical applications related thereto.

In some embodiments, such a protein is a secretagogue, for example inducing the secretion of IL-10 by human immune cells. Moreover, the protein may be immunomodulatory.

Accordingly, the human microbiota protein may be a mimic or a secretagogue of a human host protein, e.g. selected from the group consisting of cytokines, interleukins, chemokines, growth factors, neuropeptides and peptide hormones.

Exemplified human microbiota protein drug candidates were identified by the present inventors as described above and, in more detail, in the Example section. The amino acid sequences of those exemplified microbiota drug candidates are shown in the Table 1 below:

**Table 1: Sequences of exemplified microbiota proteins inducing and/or enhancing IL-10 secretion from human cells.**

| **SEQ ID NO** | **Sequence** |
|---|---|
| SEQ ID NO: 1 | AFLFTSTGVPKKAAEAAFFLYLNKGTKKGSRSCFFIYLDRGTKKG SRSCFFYLPRQGYQKGSRGCFFIYLDRGTKKGSRGCFFIYLDCE KRAGNVCIRKCRGRYLHKKTPRRYRNAEATCS |
| SEQ ID NO: 2 | QTRKQREDAKREAWKKERKEKKALEAQQDSVSFMKDTESCCA SKAFFSLRSFFHASRLASSLCFLVCAFVTPLKQTSNNAAKNNTFFI IKAVLLINISFR |
| SEQ ID NO: 3 | ARNYTCDVCGNGTIQIVSSHIIHNVHCGFIPCNKINGVMDEV VYKTVTENNEACNNCGVSYTYKVYGDMEIICKAKAN |
| SEQ ID NO: 4 | AEPADTAISERRVELCGNCGGRMVTSTTWGSWYTVAQIKCTH HNYGTDLRQQRDGTATTKCQGCGQGYTTSKSQTRIVCHGY DS |
| SEQ ID NO: 5 | AAFVFSNSLKPANASSAESSRLLIHVNSFFSQLGLKPISENLLRKT AHFCEFGMLGILASSACAMFSGAYSAASLPSLRRRGFFISFGVSV ACAVCDETIQYFVPGRACRVTDMLIDSAGALCGLAAVLAFCA AIRVRRRRRRN |
| SEQ ID NO: 6 | LAGPGSGCRFTPSCSTYFIQAVEIHGALKGSLMGIWRILRCNP WGGCGYDPVPPRKPR |
| SEQ ID NO: 7 | AKLGMAAGAMLVLGLLAAGASGGTLILAALALCAVTLLCGRK KQ |
| SEQ ID NO: 8 | VEKKTVITKCAITVNEYREKVVPSMRKIHAIVIFVSYSINHLYKNC EPEQLFSPGRKTKKPPATCRKRLNLQYF |
| SEQ ID NO: 9 | EITQPCNHVKSDWIIDKEATCIGSYAFYNCTSLTSIEISTSVTKIKY RAFASCRALNNIYYTGTLTQWNEISKDTNWNWAAPLNCKVIC LNGTCYL |
| SEQ ID NO: 10 | LLVSVCTAAGLLAVAMRQIEPLLAWLRTLEVYFQGQSPAVLLR ALGIALVAQFAADTCREAGLCAASTAIELCGRVLVLLQALPLLRS LLGSFADYLQ |

Accordingly, a human microbiota protein drug candidate comprising the sequence as set forth in SEQ ID NO: 1 is preferred. Moreover, a human microbiota protein drug candidate comprising the sequence as set forth in SEQ ID NO: 2 is also preferred. Moreover, a human microbiota protein drug candidate comprising the sequence as set forth in SEQ ID NO: 3 is also preferred. Moreover, a human microbiota protein drug candidate comprising the sequence as set forth in SEQ ID NO: 4 is also preferred. Moreover, a human microbiota protein drug candidate comprising the sequence as set forth in SEQ ID NO: 5 is also preferred. Moreover, a human microbiota protein drug candidate comprising the sequence as set forth in SEQ ID NO: 6 is also preferred. Moreover, a human microbiota protein drug candidate comprising the sequence as set forth in SEQ ID NO: 7 is also preferred. Moreover, a human microbiota protein drug candidate comprising the sequence as set forth in SEQ ID NO: 8 is also preferred. Moreover, a human microbiota protein drug candidate comprising the sequence as set forth in SEQ ID NO: 9 is also preferred. Moreover, a human microbiota protein drug candidate comprising the sequence as set forth in SEQ ID NO: 10 is also preferred. Most preferably, the human microbiota protein drug candidate comprises or consists of the sequence as set forth in SEQ ID NO: 1.

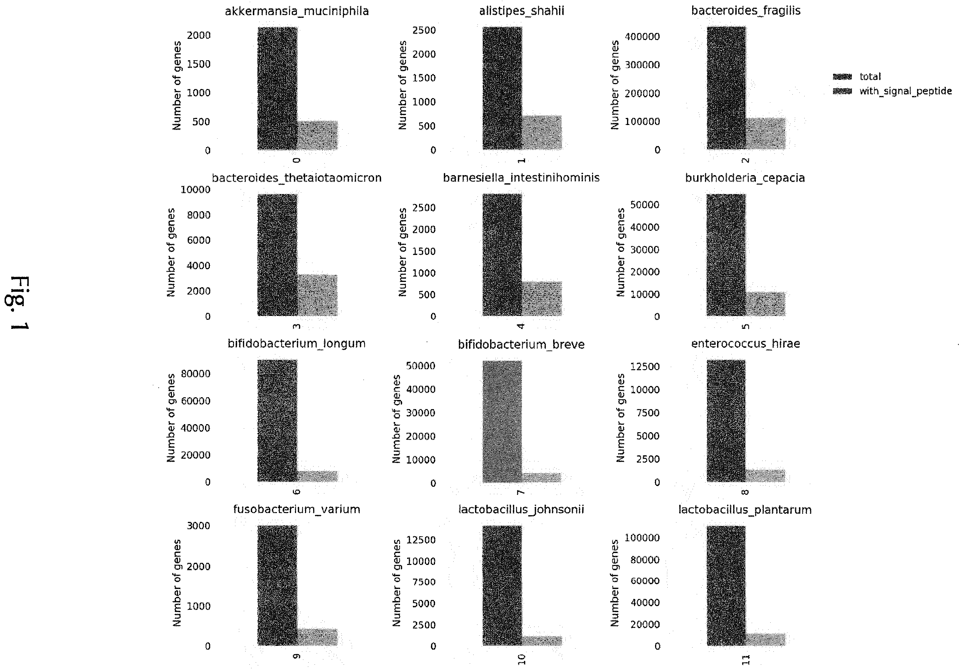

As shown in Example 1 (Fig. 1), IL-10 secretion from human cells stimulated with the exemplified microbiota proteins of SEQ ID NOs 1 - 10 is higher than IL-10 secretion from the same type of human cells stimulated with an *E. coli* lysate. Accordingly, the human microbiota drug candidates of SEQ ID NO: 1 - 10 induce and/or enhance secretion of IL-10 from human cells. Accordingly, the proteins of SEQ ID NOs 1 - 10 may be useful in the treatment of inflammatory diseases and autoimmune disorders.

Accordingly, the present invention also provides a human microbiota metasecretome protein comprising an amino acid sequence according to any one of SEQ ID NOs 1 - 10.

### Method for preparing a medicament

In a further aspect the present invention also provides a method for preparing a medicament for prevention and/or treatment of a disease comprising the following steps:
(a) providing a human microbiota protein drug candidate as described above;
(b) preparing a pharmaceutical composition comprising:
   (1) said protein of the human microbiota metasecretome or a (functional) fragment or sequence variant thereof;
   (2) a nucleic acid molecule encoding the protein according to (1);
   (3) a cell expressing the protein according to (1) or comprising the nucleic acid molecule according to (2);
   (4) an antibody binding to the protein according to (1);
   (5) a nucleic acid molecule encoding the antibody according to (4);
   (6) a cell expressing the antibody according to (4) or comprising the nucleic acid molecule according to (5);
   (7) a compound interacting with the protein according to (1); or
   (8) a compound interfering with the interaction (binding) of the protein according to (1) to the human host molecule
   and, optionally, a pharmaceutically acceptable carrier and/or an adjuvant.

In addition, the present invention provides a method for preparing a medicament for prevention and/or treatment of a disease comprising the following steps:
(a) identification of a protein of the human microbiota metasecretome interacting with a human host molecule according to the present invention as described above;
(b) preparing a pharmaceutical composition comprising:
   (1) said protein of the human microbiota metasecretome or a (functional) fragment or sequence variant thereof;
   (2) a nucleic acid molecule encoding the protein according to (1);
   (3) a cell expressing the protein according to (1) or comprising the nucleic acid molecule according to (2);
   (4) an antibody binding to the protein according to (1);
   (5) a nucleic acid molecule encoding the antibody according to (4);
   (6) a cell expressing the antibody according to (4) or comprising the nucleic acid molecule according to (5);
   (7) a compound interacting with the protein according to (1); or
   (8) a compound interfering with the interaction (binding) of the protein according to (1) to the human host molecule
   and, optionally, a pharmaceutically acceptable carrier and/or an adjuvant.

As used herein, the term "sequence variant" refers to a sequence which is similar (meaning in particular at least 50% sequence identity, see below), but not (100%) identical, to a reference sequence. Accordingly, a sequence variant contains at least one alteration in comparison to a reference sequence. In general, a sequence variant shares, in particular over the whole length of the sequence, at least 50% sequence identity with a reference sequence, whereby sequence identity can be calculated as described above. Preferably, a sequence variant shares, in particular over the whole length of the sequence, at least 60%, preferably at least 70%, more preferably at least 75%, more preferably at least 80%, even more preferably at least 85%, still more preferably at least 90%, particularly preferably at least 95%, and most preferably at least 99% sequence identity with a reference sequence (which may be calculated as described above).

In general, the term "sequence variant" includes nucleotide sequence variants and amino acid sequence variants. For example, an amino acid sequence variant has an altered sequence in which one or more of the amino acids is deleted or substituted in comparison to the reference sequence, or one or more amino acids are inserted in comparison to the reference amino acid sequence. As a result of the alterations, the amino acid sequence variant has an amino acid sequence which is at least 50%, preferably at least 60%, more preferably at least 70%, more preferably at least 75%, even more preferably at least 80%, even more preferably at least 85%, still more preferably at least 90%, particularly preferably at least 95%, most preferably at least 99% identical to the reference sequence. For example, variant sequences which are at least 90% identical have no more than 10 alterations (i.e. any combination of deletions, insertions or substitutions) per 100 amino acids of the reference sequence.

As used herein, a "fragment" of the microbiota protein comprises at least 10 consecutive amino acids of the microbiota protein, preferably at least 15 consecutive amino acids of the microbiota protein, more preferably at least 20 consecutive amino acids of the microbiota protein, even more preferably at least 25 consecutive amino acids of the microbiota protein, still more preferably at least 30 consecutive amino acids of the microbiota protein and particularly preferably at least 35 consecutive amino acids of the microbiota protein. Accordingly, the fragment of the microbiota protein has a length of at least 10 amino acids, preferably at least 15 amino acids, more preferably at least 20 amino acids, even more preferably at least 25 amino acids, still more preferably at least 30 amino acids and most preferably at least 35 amino acids. For example, the fragment of the microbiota protein may comprise 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300 or more consecutive amino acids of the microbiota protein. It is understood that the fragment of the microbiota protein is in any case shorter than the (full-length) microbiota protein (and has a minimum length of 10 amino acids).

For example, a fragment of a microbiota protein may be at least 1%, 2%, 3%, 4%, 5%, 6%, 7%, 8%, 9%, 10%, 11%, 12%, 13%, 14%, 15%, 16%, 17%, 18%, 19%, 20% or more, e.g. 25%, 30%, 35%, 40%, 45%, 50%, 55%, 60%, 65%, 70%, 75%, 80%, 85%, 90%, 95% or more, shorter than the full-length microbiota protein (representing the "reference" with 100%).

Preferably, a sequence variant or a fragment preserves the specific function of the reference sequence. In the context of the present invention, this function is the functionality of the protein of the human microbiota metasecretome, e.g. with regard to the (host) interaction and/or the biological activity as described above.

Formulation processing techniques, which are useful in the context of the preparation of medicaments, in particular pharmaceutical compositions and vaccines, according to the present invention are set out in "Part 5 of Remington's "The Science and Practice of Pharmacy", 22nd Edition, 2012, University of the Sciences in Philadelphia, Lippincott Williams & Wilkins".

As a further ingredient, the pharmaceutical composition may in particular comprise a pharmaceutically acceptable carrier and/or vehicle. In the context of the present invention, a pharmaceutically acceptable carrier typically includes the liquid or non-liquid basis of the inventive pharmaceutical composition. If the inventive pharmaceutical composition is provided in liquid form, the carrier will typically be pyrogen-free water; isotonic saline or buffered (aqueous) solutions, e.g phosphate, citrate etc. buffered solutions. Particularly for injection of the inventive pharmaceutical composition, water or preferably a buffer, more preferably an aqueous buffer, may be used, containing a sodium salt, preferably at least 30 mM of a sodium salt, a calcium salt, preferably at least 0.05 mM of a calcium salt, and optionally a potassium salt, preferably at least 1 mM of a potassium salt. According to a preferred embodiment, the sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCl, Kl, KBr, K₂CO₃, KHCO₃, K₂SO₄, and examples of calcium salts include e.g. CaCl₂, Cal₂, CaBr₂, CaCO₃, CaSO₄, Ca(OH)₂. Furthermore, organic anions of the aforementioned cations may be contained in the buffer. According to a more preferred embodiment, the buffer suitable for injection purposes as defined above, may contain salts selected from sodium chloride (NaCl), calcium chloride (CaCl₂) and optionally potassium chloride (KCl), wherein further anions may be present additional to the chlorides. CaCl₂ can also be replaced by another salt like KCl. Typically, the salts in the injection buffer are present in a concentration of at least 30 mM sodium chloride (NaCl), at least 1 mM potassium chloride (KCl) and at least 0,05 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person. Saline (0.9% NaCl) and Ringer-Lactate solution are particularly preferred as a liquid basis.

Moreover, one or more compatible solid or liquid fillers or diluents or encapsulating compounds may be used as well for the inventive pharmaceutical composition, which are suitable for administration to a subject to be treated. The term "compatible" as used herein means that these constituents of the inventive pharmaceutical composition are capable of being mixed with the protein of the human microbiota metasecretome as defined herein in such a manner that no interaction occurs which would substantially reduce the pharmaceutical effectiveness of the inventive pharmaceutical composition under typical use conditions. Pharmaceutically acceptable carriers, fillers and diluents must, of course, have sufficiently high purity and sufficiently low toxicity to make them suitable for administration to a subject to be treated. Some examples of compounds which can be used as pharmaceutically acceptable carriers, fillers or constituents thereof are sugars, such as, for example, lactose, glucose and sucrose; starches, such as, for example, corn starch or potato starch; cellulose and its derivatives, such as, for example, sodium carboxymethylcellulose, ethylcellulose, cellulose acetate; powdered tragacanth; malt; gelatin; tallow; solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol, mannitol and polyethylene glycol; alginic acid.

Further additives which may be included in the inventive pharmaceutical composition are emulsifiers, such as, for example, Tween®; wetting agents, such as, for example, sodium lauryl sulfate; colouring agents; taste-imparting agents, pharmaceutical carriers; tablet-forming agents; stabilizers; antioxidants; preservatives.

For example, the medicament may be a vaccine. As used in the context of the present invention, the term "vaccine" refers to a biological preparation that provides innate and/or adaptive immunity, typically to a particular disease. Thus, a vaccine supports in particular an innate and/or an adaptive immune response of the immune system of a subject to be treated. The vaccine may further comprise an adjuvant, which may lead to or support an innate immune response.

Accordingly, the pharmaceutical composition, in particular the vaccine, can additionally contain one or more auxiliary substances in order to further increase its immunogenicity, preferably the adjuvants described above. A synergistic action of the protein of the human microbiota metasecretome as defined above and of an auxiliary substance, which may be optionally contained in the inventive vaccine as described above, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CSF, which allow an immune response produced by the immune-stimulating adjuvant according to the invention to be enhanced and/or influenced in a targeted manner. Particularly preferred auxiliary substances are cytokines, such as monokines, lymphokines, interleukins or chemokines, that further promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IFN-alpha, IFN-beta, IFN-gamma, GM-CSF, G-CSF, M-CSF, LT-beta or TNF-alpha, growth factors, such as hGH.

The inventive pharmaceutical composition can also additionally contain any further compound, which is known to be immune-stimulating due to its binding affinity (as ligands) to human Toll-like receptors TLR1, TLR2, TLR3, TLR4, TLR5, TLR6, TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLR13.

Another class of compounds, which may be added to an inventive pharmaceutical composition, in particular to an inventive vaccine, in this context, may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences, in particular the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

Particularly preferred adjuvants are polyinosinic:polycytidylic acid (also referred to as "poly I:C") and/or its derivative poly-ICLC. Poly I:C is a mismatched double-stranded RNA with one strand being a polymer of inosinic acid, the other a polymer of cytidylic acid. Poly I:C is an immunostimulant known to interact with toll-like receptor 3 (TLR3). Poly I:C is structurally similar to double-stranded RNA, which is the "natural" stimulant of TLR3. Accordingly, poly I:C may be considered a synthetic analog of double-stranded RNA. Poly-ICLC is a synthetic complex of carboxymethylcellulose, polyinosinic-polycytidylic acid, and poly-L-lysine double-stranded RNA. Similar to poly I:C, also poly-ICLC is a ligand for TLR3. Poly I:C and poly-ICLC typically stimulate the release of cytotoxic cytokines. A preferred example of poly-ICLC is Hiltonol®.

### BRIEF DESCRIPTION OF THE FIGURES

In the following a brief description of the appended figures will be given. The figures are intended to illustrate the present invention in more detail. However, they are not intended to limit the subject matter of the invention in any way.
- Figure 1: shows for Example 1 an overview of per-species signal peptide prediction performed with Phobius.
- Figure 2: shows an overview over the workflow of an exemplified embodiment.

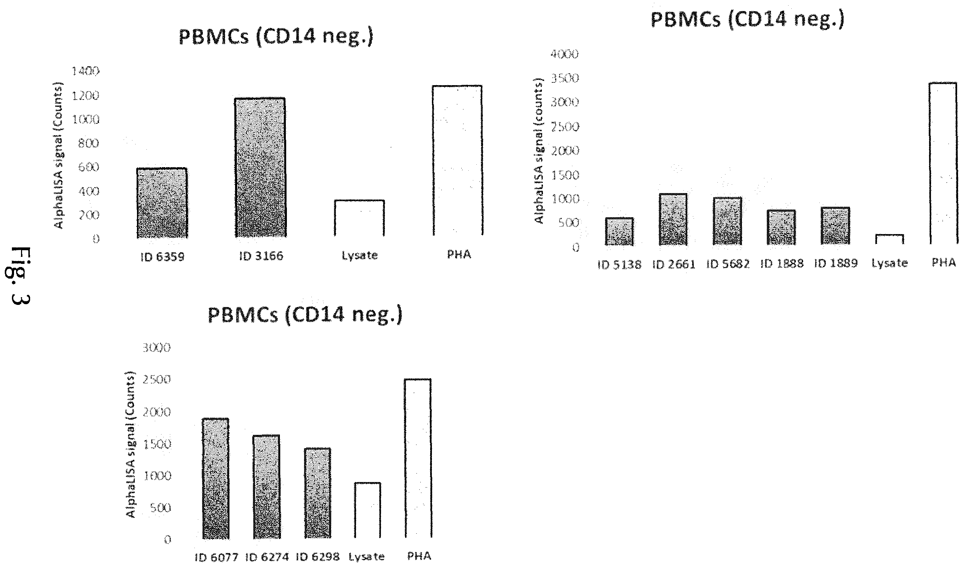

- Figure 3: shows for Example 4 the results of AlphaLISA for IL-10 secretion from human PBMCs by stimulation with 10 human microbiota protein drug candidates identified with a method according to the present invention.

### EXAMPLES

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the appended claims.

### Example 1: Identification of secreted proteins of the human microbiome

### 1. Annotation of human microbiome proteins and selection of proteins having a signal peptide

A first study was performed, wherein the microbiome genes and genomes to process and select from, were selected based on a combination of isolated bacterial genomes that are part of the human gut microbiome and known for their role in immunomodulatory activities, and genes coming from metagenomic catalogues.

A first list of species was compiled, which included twelve species selected for their role in immuno-modulation and inflammation response control:
- *Alistipes shahii*
- *Akkermansia muciniphila*
- *Bacteroides fragilis*
- *Bacteroides thetaiotaomicron*
- *Bamesiella intestinihominis*
- *Bifidobacterium breve*
- *Bifidobacterium longum*
- *Burkholderia cepacia*
- *Enterococcus hirae*
- *Fusobacterium varium*
- *Lactobacillus johnsonii*
- *Lactobacillus plantarum*

The protein and gene sequences for the 12 selected bacterial species were downloaded from the Ensembl Bacteria database (http://bacteria.ensembl.org/index.html; P.J. Kersey, J.E. Allen, A. Allot, M. Barba, S. Boddu, B.J. Bolt, D. Carvalho-Silva, M. Christensen, P. Davis, C. Grabmueller, N. Kumar, Z. Liu, T. Maurel, B. Moore, M. D. McDowall, U. Maheswari, G. Naamati, V. Newman, C.K. Ong, D.M. Bolser., N. De Silva, K.L. Howe, N. Langridge, G. Maslen, D.M. Staines, A. Yates. Ensembl Genomes 2018: an integrated omics infrastructure for non-vertebrate species Nucleic Acids Research 2018 46(D1) D802-D808).

The protein and nucleotide sequence of each gene was then imported into a MySQL database (https://www.mysql.com/; Oracle Corporation, Redwood City, USA) that was created for the study.

The total number of proteins available for each species is presented in the Table 2 below:

**Table 2: Total number of protein sequences per species, retrieved from the Ensembl Bacteria database**

| **Species** | **Count** |
|---|---|
| akkermansia_muciniphila | 2138 |
| alistipes_shahii | 2563 |
| bacteroides_fragilis | 435909 |
| bacteroides_thetaiotaomicron | 9624 |
| barnesiella_intestinihominis | 2813 |
| bifidobacterium_breve | 52264 |
| bifidobacterium_longum | 90356 |
| burkholderia_cepacia | 54695 |
| enterococcus_hirae | 13257 |
| fusobacterium_varium | 3008 |
| lactobacillus_johnsonii | 14141 |
| lactobacillus_plantarum | 110870 |

Among others, the different numbers of proteins per species are due to the numbers of genomes and strains of each species in the public database.

All 791,638 available proteins across the 12 selected species were processed using the Phobius software (A combined transmembrane topology and signal peptide predictor, Stockholm Bioinformatics Centre; URL: http://phobius.sbc.su.se/; Lukas Käll, Anders Krogh and Erik L. L. Sonnhammer. A Combined Transmembrane Topology and Signal Peptide Prediction Method. Journal of Molecular Biology, 338(5):1027-1036, May 2004; Lukas Käll, Anders Krogh and Erik L. L. Sonnhammer. Advantages of combined transmembrane topology and signal peptide prediction--the Phobius web server Nucleic Acids Res., 35:W429-32, July 2007) to predict the presence of a signal peptide. Out of the 791,638 proteins 155,336 proteins were found positive. The Phobius results for each protein were imported into the MySQL database. Figure 1 shows the number of proteins where Phobius found a signal peptide.

Next, the complete set of proteins was annotated using HMMSCAN (HmmerWeb version 2.21.0; https://www.ebi.ac.uk/Tools/hmmer/search/hmmscan; Finn, R. D., Clements, J. & Eddy, S. R. HMMER web server: interactive sequence similarity searching. Nucleic Acids Res 39, W29-37, doi:10.1093/nar/gkr367 (2011); Eddy, S. R. Accelerated Profile HMM Searches. PLoS Comput Biol 7, e1002195, doi:10.1371/journal.pcbi.1002195 (2011); R.D. Finn, J. Clements, W. Arndt, B.L. Miller, T.J. Wheeler, F. Schreiber, A. Bateman and S.R. Eddy: HMMER web server: 2015 update. Nucleic Acids Research (2015) Web Server Issue 43:W30-W38) and the PFAM database (Version 31.0; http://pfam.xfam.org/; R.D. Finn, P. Coggill, R.Y. Eberhardt, S.R. Eddy, J. Mistry, A.L. Mitchell, S.C. Potter, M. Punta, M. Qureshi, A. Sangrador-Vegas, G.A. Salazar, J. Tate, A. Bateman: The Pfam protein families database: towards a more sustainable future. Nucleic Acids Research (2016) Database Issue 44: D279-D285; E. L. Sonnhammer, S. R. Eddy, R. Durbin: Pfam: a comprehensive database of protein domain families based on seed alignments. In: Proteins. 28, 1997, S. 405-420) to facilitate a downstream filtering and selection of predicted secreted proteins. All PFAM domain results were imported into the same MySQL database along with protein sequences and Phobius predictions.

### 2. Removal of redundant proteins and length filter

To reduce redundancy within each species, a sequence clustering was performed on the subset of proteins found positive for the signal peptide prediction with Phobius using CD-HIT (http://cd-hit.org; http://weizhongli-lab.org/cd-hit/; Weizhong Li, Lukasz Jaroszewski & Adam Godzik. Clustering of highly homologous sequences to reduce the size of large protein databases. Bioinformatics (2001) 17:282-283; Weizhong Li & Adam Godzik. Cd-hit: a fast program for clustering and comparing large sets of protein or nucleotide sequences. Bioinformatics (2006) 22:1658-1659; Weizhong Li, Limin Fu, Beifang Niu, Sitao Wu and John Wooley. Ultrafast clustering algorithms for metagenomic of CD-HIT-OTU-MiSeqsequence analysis. Briefings in Bioinformatics, (2012) 13 (6): 656-668). An identity cut-off at 75% was applied. The number of non-redundant proteins per species, having a signal peptide, is reported in the Table 3 below:

**Table 3: Number of signal peptide positive and non-redundant protein sequences per species after clustering with CD-HIT**

| **Species** | **Count** |
|---|---|
| akkermansia_muciniphila | 499 |
| alistipes_shahii | 707 |
| bacteroides_fragilis | 5105 |
| bacteroides_thetaiotaomicron | 2039 |
| barnesiella_intestinihominis | 794 |
| bifidobacterium_breve | 513 |
| bifidobacterium_longum | 785 |
| burkholderia_cepacia | 3042 |
| enterococcus_hirae | 350 |
| fusobacterium_varium | 415 |
| lactobacillus_johnsonii | 287 |
| lactobacillus_plantarum | 1093 |

An additional filter was imposed on the length of the protein sequences, to select only those proteins having a length from 50 to 250 amino acids . The reason for this filtering is to avoid considering at this stage smaller peptides which can result from annotation biases or artifacts, and at the same time limit the maximum length of the selected proteins, to be best suited at this stage for the *in vitro* synthesis and the downstream laboratory tests. Furthermore, proteins larger than 250 amino acids are usually enzymes, while peptide hormones, growth factors and cytokine-like proteins are generally shorter than 250 amino acids and those types of peptides can be directly tested *in vitro* on several cellular receptors to assess their possible modulatory effects. The number of per-species proteins after the length filter is presented in the Table 4 below:

**Table 4: Number of non-redundant proteins having a signal peptide with Phobius analysis and a length between 50 and 250 amino acids.**

| **Species** | **Count** |
|---|---|
| akkermansia_muciniphila | 152 |
| alistipes_shahii | 175 |
| bacteroides_fragilis | 1529 |
| bacteroides_thetaiotaomicron | 387 |
| barnesiella_intestinihominis | 183 |
| bifidobacterium_breve | 210 |
| bifidobacterium_longum | 313 |
| burkholderia_cepacia | 1185 |
| enterococcus_hirae | 157 |
| fusobacterium_varium | 160 |
| lactobacillus_johnsonii | 104 |
| lactobacillus_plantarum | 500 |

Accordingly, a list of 5,055 proteins was compiled.

### 3. Selecting proteins having signal peptides based on a distinct algorithm and removal of incomplete/truncated sequences

An additional filtering step was performed to process the resulting protein sequences with SignalP v4.1 software (Center for biological sequence analysis, Technical University of Denmark DTU; URL: www.cbs.dtu.dk/services/SignalP; Henrik Nielsen, Jacob Engelbrecht, Søren Brunak and Gunnar von Heijne. Identification of prokaryotic and eukaryotic signal peptides and prediction of their cleavage sites. Protein Engineering, 10:1-6, 1997; Thomas Nordahl Petersen, Søren Brunak, Gunnar von Heijne & Henrik Nielsen. SignalP 4.0: discriminating signal peptides from transmembrane regions. Nature Methods, 8:785-786, 2011). Only the proteins showing a positive prediction of the signal peptide (either for the Gram+ or Gram- method used by SignalP) were retained.

Thereafter, corresponding nucleotide sequences were searched for proper start and stop codons to assure that the original gene was "complete". Thereby incomplete genes (and truncated protein sequences) were removed from the list.

The final number of protein sequences at this stage, after the above-described filtering steps and the double signal peptide prediction with Phobius and SignalP methods, included 2,573 sequences of secreted human microbiome proteins.

### Example 2: In silico cleavage of the signal peptide

For the downstream protein synthesis and analysis only the final "leaderless" proteins are relevant, for example to obtain correctly folded proteins and to correctly calculate the amino acids frequencies in each sequence. Therefore, in the above identified protein sequences the signal peptides were removed.

To this end, the cleavage site given by the "Y score" from SignalP was used. In cases where both the Gram+ and Gram- SignalP predictions were positive for the same sequence, the smaller cleavage site was considered.

### Example 3: Cysteine motifs identification

In order to identify proteins having an increased rigid structure, the final list of 2,573 proteins was searched for cysteine-rich proteins, wherein the cysteines form disulfide bonds.

To this end, KAPPA (http://kappa-sequence-search.sourceforge.net; Joly V, Matton DP. KAPPA, a simple algorithm for discovery and clustering of proteins defined by a key amino acid pattern: a case study of the cysteine-rich proteins. Bioinformatics. 2015 Jun 1;31(11):1716-23), a recently published algorithm for discovery and clustering of proteins defined by a key amino acid pattern was used to process the 2,573 sequences obtained after filtering, double signal peptide prediction and the *in silico* cleavage of the signal peptide.

With the KAPPA analysis, 25 protein clusters were identified sharing common Cysteine-motifs, the following table summarizes the results of the KAPPA run:

**Table 5: The proteins of each KAPPA cluster with the median number of cysteines per protein in each cluster.**

| **Cluster ID** | **Nb of proteins** | **Median Nb of Cys** |
|---|---|---|
| Cluster_01 | 510 | 2.0 |
| Cluster_02 | 196 | 3.0 |
| Cluster_03 | 145 | 4.0 |
| Cluster_04 | 68 | 4.0 |
| Cluster_05 | 54 | 2.0 |
| Cluster_06 | 8 | 3.0 |
| Cluster_07 | 7 | 4.0 |
| Cluster_08 | 7 | 4.0 |
| Cluster_09 | 6 | 2.0 |
| Cluster_10 | 5 | 2.0 |
| Cluster_11 | 4 | 3.0 |
| Cluster_12 | 4 | 4.0 |
| Cluster_13 | 3 | 9.0 |
| Cluster_14 | 3 | 8.0 |
| Cluster_15 | 3 | 3.0 |
| Cluster_16 | 3 | 5.0 |
| Cluster_17 | 3 | 6.0 |
| Cluster_18 | 3 | 6.0 |
| Cluster_19 | 2 | 8.0 |
| Cluster_20 | 2 | 4.0 |
| Cluster_21 | 2 | 8.0 |
| Cluster_22 | 2 | 8.0 |
| Cluster_23 | 2 | 6.0 |
| Cluster_24 | 2 | 7.0 |
| Cluster_25 | 2 | 4.0 |
| Clustering_singletons | 47 | 7.0 |

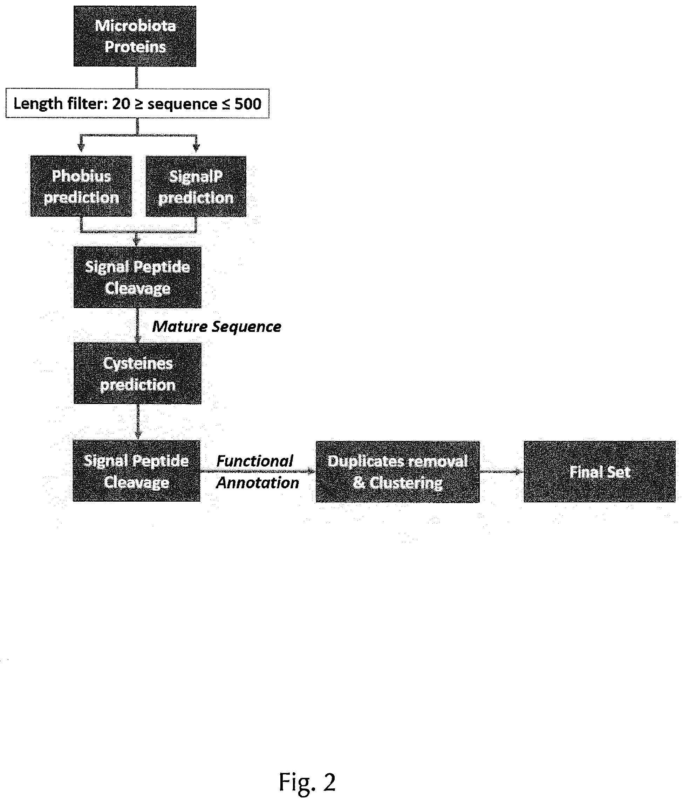

An overview over the work flow of an exemplified embodiment, in particular wherein the methods described in Examples 1 - 3 were performed, is shown in Figure 2.

The human microbiota protein drug candidates identified in Examples 1 - 3 were produced as described herein.

### Example 4: Identification of human microbiota-derived protein drug candidates inducing IL-10 release from human cells

The aim of this study was to identify proteins expressed by human microbiota, which are able to induce secretion of IL-10 from human immune cells. To this end, a library of proteins expressed by human microbiota was constructed and screened to identify proteins inducing secretion of IL-10 from human immune cells.

### Experimental Procedures:

### Library: In silico method

A compound library of secreted proteins from gut commensal bacteria was generated by an *in silico*-based approach. The library included more than 12,000 proteins predicted from human gut microbiome catalogues and from bacterial species known for their role in immune modulation. To obtain the library, bacterial proteins having a length from 50 to 350 amino acids (length of pre-proteins including the signal peptide) were screened for the presence of secretory signal peptides using the bioinformatic tool Phobius and were annotated using HMMSCAN and the PFAM database. A cut-off at 75% was applied to reduce sequence redundancy.

In view of the relevance of small cysteine-rich proteins in immune modulation an additional selection criterion was applied to identify cysteine-rich proteins: at least two cysteines were required to be present to form a disulphide bond. To ensure correct synthesis and folding *in vitro,* the amino acid sequences corresponding to the signal peptide were removed.

### Library: Cell free proteins synthesis and quantification

The protein library was generated using an *Escherichia coli* Cell Free kit suitable for generation of disulphide bonds (RTS 100 E. coli Disulfide Kit; Biotechrabbit, Hennigsdorf, Germany) according to the supplier's protocol. The Cell-Free system is based on the continuous exchange between the reaction compartment, containing components for transcription and translation, and the feeding chamber, containing amino acids and other energy components, through a semipermeable membrane.

Heterologous protein expression using the transcriptional machinery of *E. coli* was improved through a codon optimization algorithm (Twist Bioscience, San Francisco, USA) applied to all the selected sequences. All synthetized ORFs were subcloned into pIVEX 2.4 vector (Biotechrabbit, Hennigsdorf, Germany) specifically designed for high-yield Cell-Free expression of His-tagged proteins.

For the detection of His-tagged proteins, the 6His Check kit Gold using the HTRF® technology (Cisbio, Codolet, France) was used according to the supplier's protocol. Proteins, previously diluted at 1:20 in 1X PBS, were quantified in 384 well plates against a standard curve of 6xHis GFP at 0.1µg/mL (ThermoFisher, Waltham, USA) diluted in serial dilution in the lysate used for the Cell-Free synthesis (lysate was also diluted at 1:20 in 1X PBS).

### E. coli production of recombinant proteins

DNA from positive hits was subcloned in pET-28a vector carrying an N-terminal 6xHis-Tag (Twist Bioscience, San Francisco, USA) and then transformed in *E. coli* BL21(DE3) thermo competent cells (New England Biolabs, Ipswich, MA, USA). For the expression of recombinant proteins, pre-cultures of BL21(DE3) clones were performed in LB-medium at 30°C under shaking (180 rpm) conditions. Cultures were made under the same conditions and the induction was started when an OD600 of 0.4 - 0.8 was reached by using 0.5 mM IPTG. Depending on the properties of each protein, induction was performed for 2 hours to overnight.

Cultures were centrifuged for 15 min at 4°C, 4500 rpm. Supernatants were removed and the pellets were frozen at -80°C to break the cells. Pellets were then thawed and resuspended in 1X BugBuster® (Novagen®, Merck KGaA, Darmstadt, Germany) supplemented with Benzonase® Nuclease (Sigma-Aldrich, St. Louis, USA) and Lysozyme (Sigma-Aldrich, St. Louis, USA). Samples were incubated at room temperature by gentle shaking and centrifuged at 4°C, 15,000 g for 30 minutes. Soluble proteins were purified from supernatants onto Nickel packed columns (Protino®, Macherey-Nagel, Düren, Germany) according to the supplier's protocol. Proteins produced in inclusion bodies were solubilized from pellets using 8M urea. Imidazole (250 mM), used for proteins elution, was removed by buffer exchange using 3kDa cutoff filters (Amicon®, Merck Millipore Ltd., Burlington, USA). Proteins were visualized on 12% Bis-Tris acrylamide gels (ThermoFisher, Waltham, USA) stained with Coomassie Blue (Imperial protein Stain; ThermoFisher, Waltham, USA) and detected by Western Blot using the 6X-His Tag monoclonal antibody HIS.H8 (ThermoFisher, Waltham, USA) diluted at 1 :3000 and revealed using the secondary antibody Goat anti-Mouse IgG H+L WesternDot625 (ThermoFisher, Waltham, USA). Purified proteins were quantified by Bradford protein assay (Bradford M (1976) A rapid and sensitive method for the quantitation of microgram quantities of protein utilizing the principle of protein-dye binding. Anal. Biochem. 72:248-254).

### IL10 screening

IL-10 screening of the microbiota protein library was performed on CD14 depleted human peripheral blood mononuclear cells (PBMCs). This cellular model was chosen to reduce the background due to cell wall components and other bacterial contaminants possibly present in the lysate of the Cell Free synthesis kit on the synthesised (but not purified) proteins.

### CD14-depleted PBMCs:

PBMCs were isolated from buffy coats as follows: 80 ml PBS were added to 50 ml blood; 4 SepMate™-50 IVD tubes (Stemcell Technologies, Vancouver, Canada) were filled with 15 mL of Ficoll® (Ficoll® Paque Plus; Sigma-Aldrich, St. Louis, USA) per donor, then 30 ml of PBS-diluted blood were gently added. Samples were centrifuged for 20 min at 1200 g at room temperature and washed three times with PBS. To lyse the red blood cells, pellets were resuspended in Red Blood Cells Lysis buffer 1X (Miltenyi Biotec, Bergisch Gladbach, Germany) and incubated for 10 min at room temperature. Cells were then washed with MACS buffer and counted.

PBMC depletion was performed using the CD14 Microbeads kit (Miltenyi Biotec, Bergisch Gladbach, Germany) according to the supplier's protocol. Depleted monocytes were resuspended in Iscove's Modified Dulbecco's Medium (IMDM; GIBCO™, Life Technologies, Carlsbad, USA) supplemented with 1 % L-Glutamine (Sigma-Aldrich, St. Louis, USA), 1 % Penicillin-Streptomycin (Sigma-Aldrich, St. Louis, USA) and 10 % of heat-inactivated FBS (Sigma-Aldrich, St. Louis, USA).

### IL-10 screening:

Screening was performed in 384 well plates in a final volume of 60 µl. PBMCs were seeded at 72,000 cells/well by multidrop (Hamilton Robotics, Martinsried, Germany) and stimulated for 72 hours with 10% (vol/vol) of the library (proteins) previously diluted at 1:10 with PBS in a humidified 5% CO₂ atmosphere at 37°C. The *E*. *coli* lysate included in the cell free kit was used (at the same dilution as the library) as negative control. Phytohaemagglutinin (PHA) at 10 µg/ml (0.087 µM) was used as positive control. To ensure technical robustness, the screening was performed on CD14 depleted PBMCs from at least two different donors.

IL-10 secretion was measured by AlphaLISA® (IL10 (human) AlphaLISA Detection Kit; PerkinElmer, Waltham MA, USA) on the undiluted supernatants according to the supplier's protocol. Results were expressed as AlphaLISA signal (Counts). Results were considered as positive hits, either when at least a single raw data signal (of the two signals of the two PBMC donors) was higher than the corresponding plate mean + 3SD (Standard Deviation) or when both raw data signals (of the two signals of the two PBMC donors) were higher than the corresponding plate means + 2SD (Standard Deviation). In order to avoid false positives, the concentration of the potential hits obtained by the primary screening was compared to that of the corresponding plate mean.

Potential hits were then validated by a new round of Cell-Free synthesis and test on CD14 depleted PBMCs from several donors (as described above). Further characterization was performed on recombinant proteins produced in the *E*. *coli* BL21 (DE3) strain transformed with a pET-28a vector containing the target sequence (as described above, see paragraph "E. coli production of recombinant proteins"). Alternatively, a cell-free production of some proteins was performed by a commercial supplier (Synthelis, La Tronche, France).

### Results

### Screening

A total of 11904 proteins of the library was screened on CD14 depleted PBMCs in order to identify proteins able to stimulate IL-10 secretion from human PBMCs. From various potential hits obtained in the primary screening, so far ten proteins were confirmed in the second round of cell-free synthesis. These microbiota proteins, which are able to stimulate IL-10 secretion from human PBMCs, include ID3166 (SEQ ID NO: 1); ID6359 (SEQ ID NO: 2); ID1888 (SEQ ID NO: 3); ID1889 (SEQ ID NO: 4); ID2661 (SEQ ID NO: 5); ID5682 (SEQ ID NO: 6); ID5138 (SEQ ID NO: 7); ID6077 (SEQ ID NO: 8); ID6274 (SEQ ID NO: 9); and ID6298 (SEQ ID NO: 10).

Results of the AlphaLISA for IL-10 secretion from human PBMCs of the second round of cell-free synthesis are shown in Figure 1. The data show that ten IL-10 secretagogue proteins were identified by screening of a human microbiome metasecretome protein library on stimulation of IL-10 release from human PBMCs. These microbiota proteins induce IL-10 release from human immune cells.

**TABLE OF SEQUENCES AND SEQ ID NUMBERS (SEQUENCE LISTING):**

| **SEQ ID NO** | **Sequence** | **Remarks** |
|---|---|---|
| SEQ ID NO: 1 | AFLFTSTGVPKKAAEAAFFLYLNKGTKKGSRSCFFIY LDRGTKKGSRSCFFYLPRQGYQKGSRGCFFIYLDR GTKKGSRGCFFIYLDCEKRAGNVCIRKCRGRYLHK KTPRRYRNAEATCS | ID3166 |
| SEQ ID NO: 2 | QTRKQREDAKREAWKKERKEKKALEAQQDSVSF MKDTESCCASKAFFSLRSFFHASRLASSLCFLVCAFV TPLKQTSNNAAKNNTFFIIKAVLLINISFR | ID6359 |
| SEQ ID NO: 3 | ARNYTCDVCGNGTIQIVSSHIIHNVHCGFIPCNKI NGVMDEVVYKTVTENNEACNNCGVSYTYKVYG DMEIICKAKAN | D1888 |
| SEQ ID NO: 4 | AEPADTAISERRVELCGNCGGRMVTSTTWGSWYT VAQIKCTHHNYGTDLRQQRDGTATTKCQGCGQ GYTTSKSQTRIVCHGYDS | D1889 |
| SEQ ID NO: 5 | AAFVFSNSLKPANASSAESSRLLIHVNSFFSQLGLKP ISENLLRKTAHFCEFGMLGILASSACAMFSGAYSAA SLPSLRRRGFFISFGVSVACAVCDETIQYFVPGRAC RVTDMLIDSAGALCGLAAVLAFCAAIRVRRRRRRN | D2661 |
| SEQ ID NO: 6 | LAGPGSGCRFTPSCSTYFIQAVEIHGALKGSLMGI WRILRCNPWGGCGYDPVPPRKPR | ID5682 |
| SEQ ID NO: 7 | AKLGMAAGAMLVLGLLAAGASGGTLILAALALCA VTLLCGRKKQ | D5138 |
| SEQ ID NO: 8 | VEKKTVITKCAITVNEYREKVVPSMRKIHAIVIFVSYS INHLYKNCEPEQLFSPGRKTKKPPPATCRKRLNLQ YF | D6077 |
| SEQ ID NO: 9 | EITQPCNHVKSDWIIDKEATCIGSYAFYNCTSLTSIE ISTSVTKIKYRAFASCRALNNIYYTGTLTQWNEISK DTNWNWAAPLNCKVICLNGTCYL | D6274 |
| SEQ ID NO: 10 | LLVSVCTAAGLLAVAMRQIEPLLAWLRTLEVYFQG QSPAVLLRALGIALVAQFAADTCREAGLCAASTAIE LCGRVLVLLQALPLLRSLLGSFADYLQ | D6298 |

## Claims

1. A method for providing a human microbiota protein drug candidate, the method comprising the following steps:
(i) providing a plurality of human microbiota protein sequences and/or a plurality of nucleic acid sequences encoding a plurality of human microbiota proteins;
(ii) identifying in the sequences provided in step (i) one or more sequence(s) of (a) human microbiota protein drug candidate(s), wherein the sequence(s) of the human microbiota protein drug candidate(s) is/are selected according to the following criteria:
- (a) sequence(s) having, or coding for proteins having, a signal peptide;
- (a) sequence(s) having, or coding for proteins having, a length of 20 - 500 amino acids; and
- (a) sequence(s) comprising, or coding for (a) protein(s) comprising, at least two cysteine residues and/or a primary and/or a secondary structure element conferring a conformational rigid structure; and
(iii) preparing one or more human microbiota protein(s) having, or encoded by, the sequence(s) identified in step (ii).

2. The method according to claim 1, wherein the human microbiota protein is a bacterial protein of the human gastrointestinal tract microbiota.

3. The method according to claim 1 or 2, wherein the human microbiota protein has a length of 20 - 350 amino acids.

4. The method according to any one of claims 1 to 3, wherein the human microbiota protein contains an even number of cysteine residues, e.g. forming at least one or two cysteine pairs.

5. The method according to any one of claims 1 to 4, wherein the primary and/or a secondary structure element conferring a conformational rigid structure is selected from the group consisting of disulfide bridge, leucine-rich repeat, alpha-helix, beta-sheet and coil.

6. The method according to any one of claims 1 to 5, wherein the human microbiota protein has a length of 20 - 200 amino acids and comprises at least two cysteine residues, e.g. forming at least one cysteine pair.

7. The method according to any one of claims 1 to 6, wherein the human microbiota protein has a length of 50 - 150 amino acids and comprises at least four cysteine residues, e.g. forming at least two cysteine pairs.

8. The method according to any one of claims 1 to 6, wherein the human microbiota protein has a length of 20 - 50 amino acids and comprises a secondary structure element selected from the group consisting of an alpha-helix, a beta-sheet and a coil.

9. The method according to any one claims 1 to 8, wherein the human microbiota protein is a mimic or a secretagogue of a human host protein, e.g. selected from the group consisting of cytokines, interleukins, chemokines, growth factors, neuropeptides and peptide hormones.

10. The method according to claim 9, wherein the human microbiota protein is a secretagogue inducing secretion of Interleukin-10 (IL-10) by human immune cells.

11. The method according to any one of claims 1 to 10, wherein the identified protein is immunomodulatory.

12. The method according to any one of claims 1 to 11, wherein the method comprises an additional step (iv) of determining at least one biological activity of the obtained protein(s), in particular relating to an interaction with the human host.

13. The method according to claim 12, wherein the structure of the human microbiota protein is determined and compared to the structure of a human host protein.

14. The method according to any one of claims 12 or 13, wherein the obtained protein is tested using an assay relating to immunity, optionally involving human immune cells, such as peripheral blood mononuclear cells (PBMCs) or a selected subset of human immune cells.

15. A library of human microbiota protein drug candidates obtainable by the method according to any one claims 1 to 14.
